# EUROPEAN PATENT SPECIFICATION

(11) **EP 0 789 767 B1**
(45) Date of publication and mention of the grant of the patent: **16.07.2003**
(21) Application number: 95942431.8
(22) Date of filing: 13.11.1995
(51) Int. Cl.: C12N 15/37, C07K 14/025, A61K 39/12, G01N 33/569

(54) **A method for producing purified papillomavirus proteins**
Verfahren zur Herstellung von gereinigten papillomavirus Proteinen
Procédé d' obtention de protéines purifiées du papillomavirus

(30) Priority: 14.11.1994 US 339368
(43) Date of publication of application: 20.08.1997
(73) Proprietor: MERCK & CO., INC., Rahway, New Jersey 07065 (US)
(72) Inventor: LEHMAN, Ernest D., Rahway, NJ 07065 (US); COOK, James C., III, Rahway, NJ 07065 (US); GEORGE, Hugh A., Rahway, NJ 07065 (US); HOFMANN, Kathryn J., Rahway, NJ 07065 (US); JANSEN, Kathrin U., Rahway, NJ 07065 (US); JOYCE, Joseph G., Rahway, NJ 07065 (US); MARKUS, Henry Z., Rahway, NJ 07065 (US); SCHULTZ, Loren D., Rahway, NJ 07065 (US)
(74) Representative: Hiscock, Ian James
(86) International application number: US9515027
(87) International publication number: WO96015247

(56) References cited:
- WO-A-93/02184
- WO-A-94/05792
- WO-A-94/20137
- WO-A-95/31532
- VIROLOGY, vol. 209, no. 2, 1 June 1995 ORLANDO US, pages 506-518, K.J.HOFMANN ET AL. 'Sequence determination of human papillomavirus type 6a and assembly of virus-like particles in Saccharomyces cerevisiae'
- ROSE R.C. ET AL: 'Expression of human papillomavirus type 11 L1 protein in insect cells: In vivo and in vitro assembly of viruslike particles' J.VIROL. vol. 67, no. 4, 1993, pages 1936 - 1944

## Description

### HELD OF THE INVENTION

The present invention is directed to methods of making purified recombinant proteins of papillomavirus (PV).

### BRIEF DESCRIPTION OF THE DRAWINGS

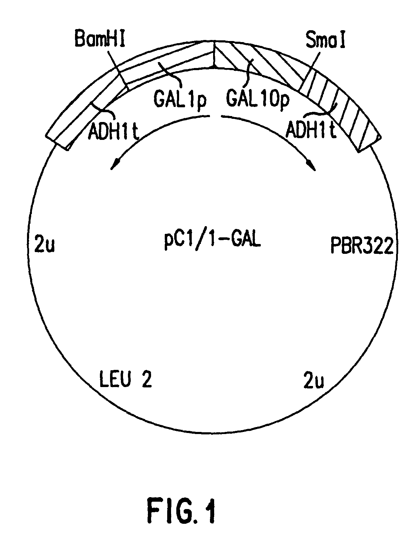

Figure 1 shows bidirectional yeast expression vector used to express papillomavirus L1 and/or L2 capsid proteins.

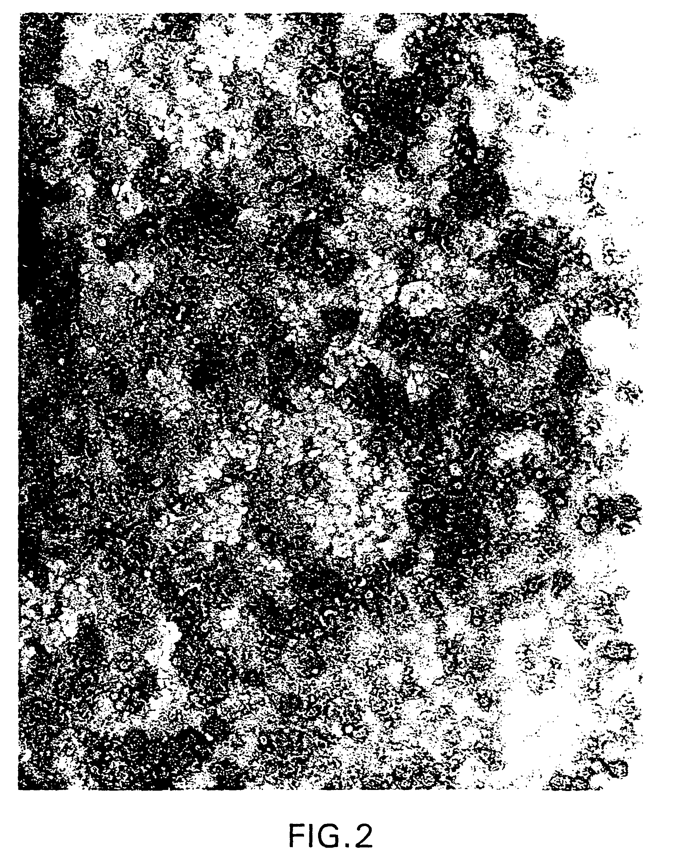

Figure 2 is an electron micrograph of HPV6a L1 VLPs expressed in yeast.

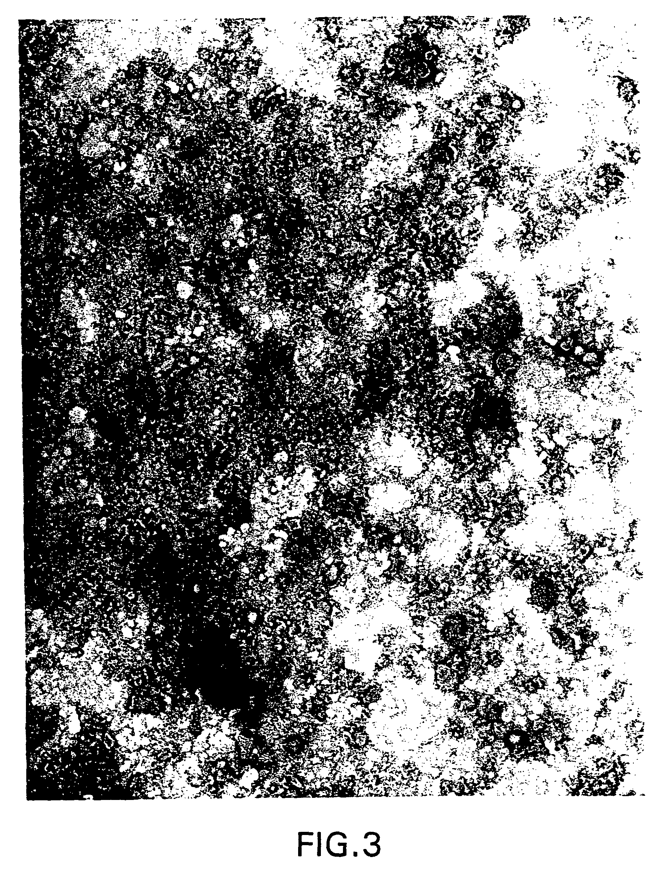

Figure 3 is an electron micrograph of HPV6a L1/L2 VLPs expressed in yeast.

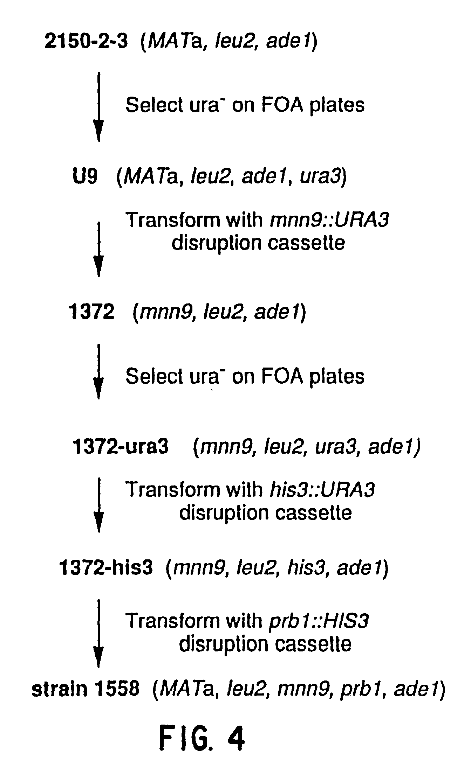

Figure 4 is a schematic overview of the construction of S. cerevisiae strain 1558.

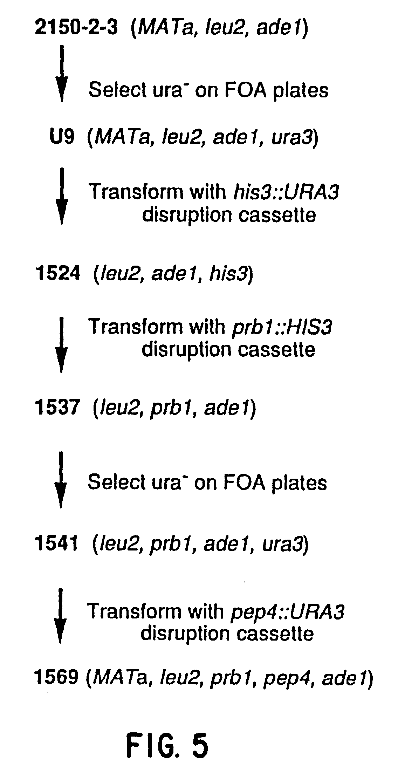

Figure 5 is a schematic overview of the construction of S. cerevisiae strain 1569.
Figure 6 outlines major steps in a purification procedure.

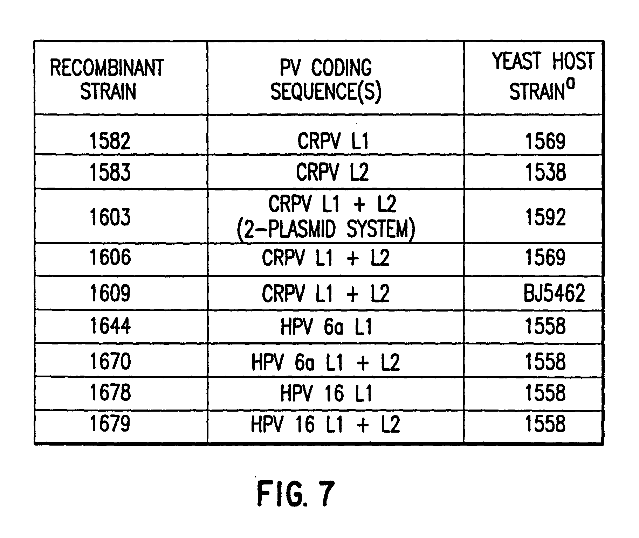

Figure 7 is list of strains.

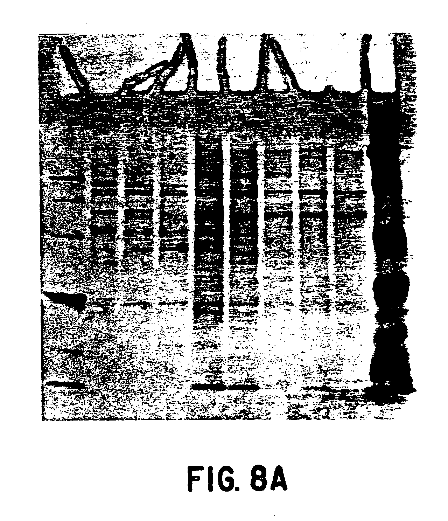

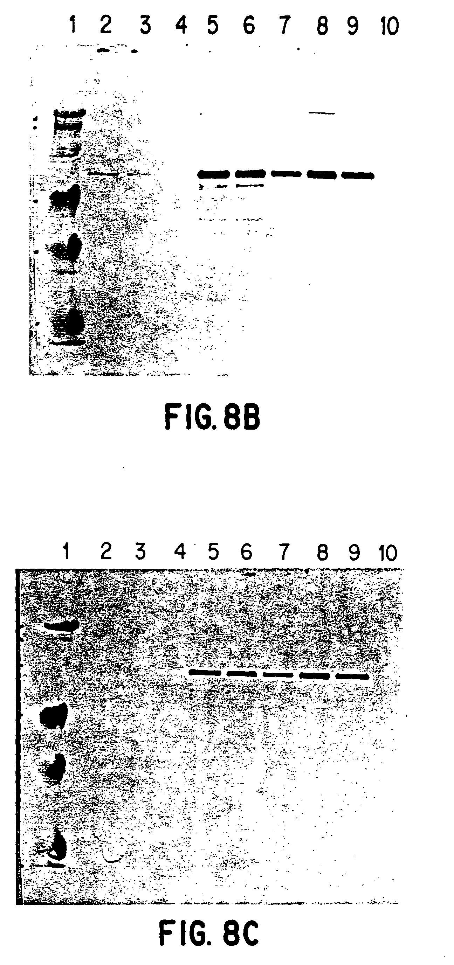

Figure 8 shows a set of SDS-PAGE analyses of HPV16 L1+L2 purified from yeast. In addition to the final purified VLP, retains from intermediate steps in the purification process are included. The table provides identification of the sample in each lane. A gel stained with colloidal Coomassie as well as Western blots probed with antisera to L1 and L2 proteins are included.

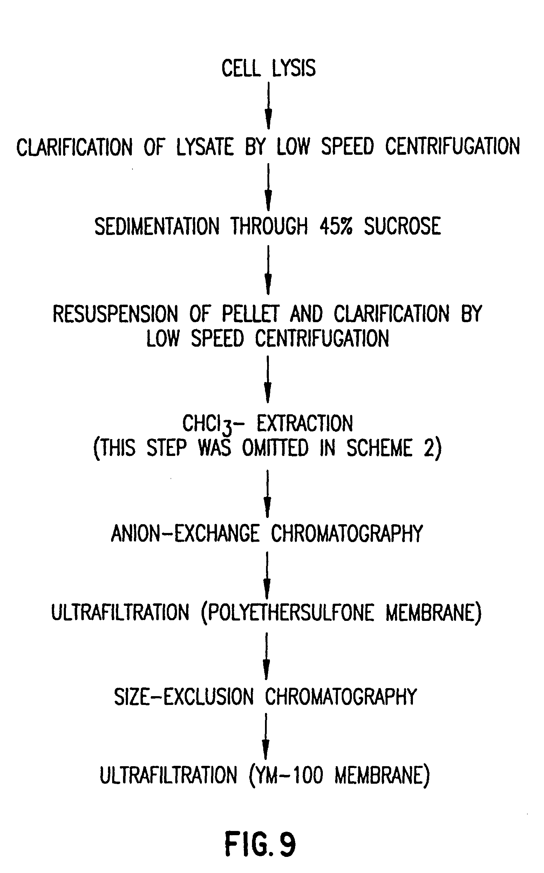

Figure 9 is a schematic of alternative cottontail rabbit papillomavirus L1 purification processes.

### BACKGROUND OF THE INVENTION

Papillomavirus (PV) infections occur in a variety of animals, including humans, sheep, dogs, cats, rabbits, monkeys, snakes and cows. Papillomaviruses infect epithelial cells, generally inducing benign epithelial or fibroepithelial tumors at the site of infection. PV are species specific infective agents; a human papillomavirus cannot infect a nonhuman animal.

Papillomaviruses may be classified into distinct groups based on the host that they infect. Human papillomaviruses (HPV) are further classified into more than 70 types based on DNA sequence homology. PV types appear to be type-specific immunogens in that a neutralizing immunity to infection by one type of papillomavirus does not confer immunity against another type of papillomavirus.

In humans, different HPV types cause distinct diseases. HPV types 1, 2, 3, 4, 7, 10 and 26-29 cause benign warts in both normal and immunocompromised individuals. HPV types 5, 8, 9, 12, 14, 15, 17, 19-25, 36 and 46-50 cause flat lesions in immunocompromised individuals. HPV types 6, 11, 34, 39, 41-44 and 51-55 cause nonmalignant condylomata of the genital or respiratory mucosa. HPV types 16, 18, 31, 33, 35, 45, and 58 cause epithelial dysplasia of the genital mucosa and are associated with the majority of in situ and invasive carcinomas of the cervix, vagina, vulva and anal canal.

Papillomaviruses are small (50-60 nm), nonenveloped, icosahedral DNA viruses that encode for up to eight early and two late genes. The open reading frames (ORFs) of the virus genomes are designated E1 to E7 and L1 and L2, where "E" denotes early and "L" denotes late. L1 and L2 code for virus capsid proteins. The early (E) genes are associated with functions such as viral replication and cellular transformation.

The L1 protein is the major capsid protein and has a molecular weight of 55-60 kDa. L2 protein is a minor capsid protein which has a predicted molecular weight of 55-60 kDa and an apparent molecular weight of 75-100 kDa as determined by polyacrylamide gel electrophoresis. Immunological data suggest that most of the L2 protein is internal to the L1 protein. The L1 ORF is highly conserved among different papillomaviruses.The L2 proteins are less conserved among different papillomaviruses.

The L1 and L2 genes have been identified as good targets for immunoprophylactics. Some of the early genes have also been demonstrated to be potential targets of vaccine development. Studies in the cottontail rabbit papillomavirus (CRPV) and bovine papillomavirus (BPV) systems have shown that immunizations with these proteins expressed in bacteria or by using vaccinia vectors protected animals from viral infection. Expression of papillomavirus L1 genes in baculovirus expression systems or using vaccinia vectors resulted in the assembly of virus-like particles (VLP) which have been used to induce high-titer virus-neutralizing antibody responses that correlate with protection from viral challenge. Furthermore, the L1 and L2 genes have been used to generate vaccines for the prevention and treatment of papillomavirus infections in animals. HPV type 16 L1 and L2 genes have been cloned into a vaccinia virus vector; the resulting vector was used to infect CV-1 mammalian cells to produce virus-like particles (VLP).

The development and commercialization of prophylactic and therapeutic vaccines for PV infection and disease containing L1 protein, L1 + L2 proteins, or modified L1 or L1 + L2 proteins has been hindered by the lack of large quantities of purified virus and purified protein. Because PV is not readily cultivated *in vitro,* it is difficult to produce the required amounts of L1 and L2 protein by *in vitro* propagation of PV. The resultant supply problems make it difficult to characterize PV and PV proteins. Accordingly, it would be useful to develop a readily renewable source of crude PV proteins, especially PV L1 and L2 proteins or modified L1 and L2 proteins. It would also be useful to develop methods of purifying large quantities of the crude papillomavirus proteins to levels of purity suitable for immunological studies and vaccine development. It would also be useful to produce large quantities of papillomavirus proteins having the immunity-conferring properties of the native proteins, such as the conformation of the native protein. In addition, it would be useful to develop methods of analyzing the PV proteins and methods of determining the relative purity of the proteins as well as compositions containing the proteins. Such highly purified proteins would also be useful in the preparation of a variety of reagents useful in the study of PV infection; such reagents include but are not limited to polyclonal antibodies, monoclonal antibodies, and analytical standards.

R.C. Rose et al., J. Virol. vol. 67, p. 1936 - 1944 (1993) disclose the purification of recombinant HPV L1 protein in two consecutive chromatographie steps.

The present invention is directed to highly purified L1 and L2 proteins of PV. The invention also comprises methods by which recombinant papillomavirus proteins having the immunity conferring properties of the native papillomavirus proteins are produced and purified. The present invention is directed to the production of prophylactic and therapeutic vaccines for papillomavirus infection. The recombinant proteins of the present invention are capable of forming virus-like particles. These VLP are immunogenic and prevent formation of warts in an animal model. The invention is exemplified with recombinant yeast-derived L1 and L 1 + L2 proteins of cottontail rabbit papillomavirus (CRPV), HPV type 6 (subtype 6a) and HPV type 16 as model systems. The purification and analytical methods of the instant application may be adapted to other HPV types, other HPV proteins and other recombinant expression systems.

### SUMMARY OF THE INVENTION

The present invention is directed to purified recombinant proteins of papillomaviruses (PV) and methods of making, measuring, using and formulating the same.

### DETAILED DESCRIPTION OF THE INVENTION

The present invention is directed to a method of producing a purified papillomavirus capsid protein comprising:
(a) transforming a yeast host with a recombinant DNA molecule, the recombinant DNA molecule encoding a protein selected from papillomavirus L1 protein and papillomavirus L1 + L2 proteins;
(b) cultivating the transformed host under conditions that permit expression of the recombinant DNA molecule to produce a crude recombinant papillomavirus protein; and
(c) purifying the crude recombinant papillomavirus protein with a single chromatography step using ion exchange chromatography to produce the purified protein, the protein being at least 90% pure.

Bacterially-derived recombinant bovine papillomavirus L1 and L2 have been generated. Neutralizing sera to the recombinant bacterial proteins cross-reacted with native virus at low levels, presumably due to differences in the conformations of the native and bacterially-derived proteins.

Recombinant baculoviruses expressing HPV16 L1 or HPV16 L2 ORFs have been used to infect insect SF9 cells and produce L1 and L2 proteins. Western blot analyses showed that the baculovirus-derived L1 and L2 proteins reacted with antibody to HPV16. The baculovirus-derived L1 forms VLPs.

The production of crude HPV16 L1 and HPV16 L2 proteins by recombinant strains of Saccharomyces cerevisiae has been reported. The recombinant proteins were produced as intracellular and as secreted products. When the proteins were expressed intracellularly, the majority of the protein was found to be insoluble when the cells were lysed in the absence of denaturing reagents. The purity of these proteins was not reported.

Recombinant proteins secreted from yeast were shown to contain yeast-derived carbohydrates. The presence of these N-linked oligosaccharides may mask native epitopes. In addition, the secreted recombinant proteins may contain other modifications, such as retention of the secretory leader sequence. The secretion process may also be less efficient.

The development and commercialization of prophylactic and therapeutic vaccines for PV infection and disease containing L1 protein, L1 + L2 proteins, or modified L1 or L1 + L2 proteins has been hindered by the lack of large quantities of purified virus and purified protein. Because PV is not readily cultivated *in vitro,* it is difficult to produce the required amounts of L1 and L2 protein by *in vitro* propagation of PV. The difficulties associated with *in vitro* cultivation of PV also result in difficulties in chemical, immunological and biological characterization of PV and PV proteins. Accordingly, it would be useful to develop a readily renewable source of crude PV proteins, especially PV L1 and L2 proteins or modified L1 and L2 proteins. It would also be useful to develop methods of purifying large quantities of the crude papillomavirus proteins to levels of purity suitable for immunological studies and vaccine development. It would also be useful to produce large quantities of papillomavirus proteins having the immunity-conferring properties of the native proteins, such as the conformation of the native protein. In addition, it would be useful to develop methods of analyzing the PV proteins and methods of determining the relative purity of the proteins as well as compositions containing the proteins. Such highly purified proteins would also be useful in the preparation of a variety of reagents useful in the study of PV infection; such reagents include but are not limited to polyclonal antibodies, monoclonal antibodies, and analytical standards.

Pharmaceutically useful compositions comprising the DNA or proteins encoded by the DNA may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the protein or VLP. Such compositions may contain proteins or VLP derived from more than one type of HPV.

Therapeutic or diagnostic compositions are administered to an individual in amounts sufficient to treat or diagnose PV infections. The effective amount may vary according to a variety of factors such as the individual's condition, weight, sex and age. Other factors include the mode of administration. Generally, the compositions will be administered in dosages ranging from about 1 mcg to about 1 mg.

The pharmaceutical compositions may be provided to the individual by a variety of routes such as subcutaneous, topical, oral, mucosal, intravenous and intramuscular.

The vaccines comprise DNA, RNA or proteins encoded by the DNA that contain the antigenic determinants necessary to induce the formation of neutralizing antibodies in the host. Such vaccines are also safe enough to be administered without danger of clinical infection; do not have toxic side effects; can be administered by an effective route; are stable; and are compatible with vaccine carriers.

The vaccines may be administered by a variety of routes, such as orally, parenterally, subcutaneously, mucosally, intravenously or intramuscularly. The dosage administered may vary with the condition, sex, weight, and age of the individual; the route of administration; and the type PV of the vaccine. The vaccine may be used in dosage forms such as capsules, suspensions, elixirs, or liquid solutions. The vaccine may be formulated with an immunologically acceptable carrier.

The vaccines are administered in therapeutically effective amounts, that is, in amounts sufficient to generate a immunologically protective response. The therapeutically effective amount may vary according to the type of PV. The vaccine may be administered in single or multiple doses.

The purified proteins of the present invention may be used in the formulation of immunogenic compositions. Such compositions, when introduced into a suitable host, are capable of inducing an immune response in the host.

The purified proteins of the invention thereof may be used to generate antibodies. The term "antibody" as used herein includes both polyclonal and monoclonal antibodies, as well as fragments thereof, such as, Fv, Fab and F(ab)2 fragments that are capable of binding antigen or hapten.

The proteins and protein derivatives of the present invention may be used to serotype HPV infection and HPV screening. The purified proteins, VLP and antibodies lend themselves to the formulation of kits suitable for the detection and serotyping of HPV. Such a kit would comprise a compartmentalized carrier suitable to hold in close confinement at least one container. The carrier may further comprise reagents such as recombinant HPV6a L1 or L2 proteins or VLP or anti-HPV6a antibodies or recombinant HPV16 L1 or L2 proteins or VLP or anti-HPV16 antibodies suitable for detecting a variety of HPV types. The carrier may also contain means for detection such as labeled antigen or enzyme substrates or the like.

The purified proteins are also useful as immunological standards, molecular weight markers and molecular size markers.

Because the genetic code is degenerate, more than one codon may be used to encode a particular amino acid, and therefore, the amino acid sequence can be encoded by any of a set of similar DNA oligonucleotides. Only one member of the set will be identical to the HPV6a or HPV 16 sequences but will be capable of hybridizing to HPV6a or HPV16 DNA even in the presence of DNA oligonucleotides with mismatches under appropriate conditions. Under alternate conditions, the mismatched DNA oligonucleotides may still hybridize to HPV6a or HPV16 DNA to permit identification and isolation of HPV6a or HPV16 encoding DNA.

The purified HPV6a or HPV16 DNA of the invention may be used to isolate and purify homologues and fragments of HPV6a from other sources. To accomplish this, the first HPV6a DNA may be mixed with a sample containing DNA encoding homologues of HPV6a under appropriate hybridization conditions. The hybridized DNA complex may be isolated and the DNA encoding the homologous DNA may be purified therefrom.

It is known that there is a substantial amount of redundancy in the various codons which code for specific amino acids. Therefore, this invention is also directed to those DNA sequences which contain alternative codons which code for the eventual translation of the identical amino acid. For purposes of this specification, a sequence bearing one or more replaced codons will be defined as a degenerate variation. Also included are mutations either in the DNA sequence or the translated protein which do not substantially alter the ultimate physical properties of the expressed protein. For example, substitution of valine for leucine, arginine for lysine, or asparagine for glutamine may not cause a change in functionality of the polypeptide.

It is known that DNA sequences coding for a peptide may be altered so as to code for a peptide having properties that are different than those of the naturally-occurring peptide. Methods of altering the DNA sequences include, but are not limited to site directed mutagenesis.

A variety of procedures known in the art may be used to molecularly clone HPV6a DNA. These methods include, but are not limited to, direct functional expression of the HPV6a genes following the construction of a HPV6a-containing cDNA or genomic DNA library in an appropriate expression vector system. Another method is to screen HPV6a-containing cDNA or genomic DNA library constructed in a bacteriophage or plasmid shuttle vector with a labeled oligonucleotide probe designed from the amino acid sequence of the HPV6a. An additional method consists of screening a HPV6a-containing cDNA or genomic DNA library constructed in a bacteriophage or plasmid shuttle vector with a partial DNA encoding the HPV6a. This partial DNA is obtained by the specific polymerase chain reaction (PCR) amplification of HPV6a DNA fragments through the design of degenerate oligonucleotide primers from the amino acid sequence of purified HPV6a. Another method is to isolate RNA from HPV6a-producing cells and translate the RNA into protein via an *in vitro* or an *in vivo* translation system. The translation of the RNA into a peptide or a protein will result in the production of at least a portion of HPV6a protein which can be identified by, for example, the activity of HPV6a protein or by immunological reactivity with an anti-HPV6a antibody. In this method, pools of RNA isolated from HPV6a-producing cells can be analyzed for the presence of an RNA which encodes at least a portion of the HPV6a. Further fractionation of the RNA pool can be done to purify the HPV6a RNA from non-HPV6a RNA. The peptide or protein produced by this method may be analyzed to provide amino acid sequences which in turn are used to provide primers for production of HPV6a cDNA, or the RNA used for translation can be analyzed to provide nucleotide sequences encoding HPV6a and produce probes for the screening of a HPV6a cDNA library. These methods are known in the art and can be found in, for example, Sambrook, J., Fritsch, E.F., Maniatis, T. in Molecular Cloning: A Laboratory Manual, Second Edition, Cold Spring Harbor Laboratory Press, Cold Spring Harbor, NY. 1989.

It is apparent to those skilled in the art that other types of libraries, as well as libraries constructed from other cells or cell types, may be useful for isolating HPV6a-encoding DNA. Other types of libraries include, but are not limited to, cDNA libraries derived from other cells or cell lines containing HPV type 6a and genomic DNA libraries.

Preparation of cDNA libraries can be performed by standard techniques well known in the art. cDNA library construction techniques can be found for example, Sambrook, J., et al., supra. It is apparent to those skilled in the art that DNA encoding HPV6a may also be isolated from a suitable genomic DNA library. Construction of genomic DNA libraries can be performed by standard techniques known in the art. Genomic DNA library construction techniques can be found in Sambrook, J., et al. supra.

The cloned HPV6a DNA or fragments thereof obtained through the methods described herein may be recombinantly expressed by molecular cloning into an expression vector containing a suitable promoter and other appropriate transcription regulatory elements, and transferred into eukaryotic host cells to produce recombinant HPV6a. Techniques for such manipulations are fully described in Sambrook, J., et al., supra, and are known in the art.

Expression vectors are defined herein as DNA sequences that are required for the transcription of cloned copies of genes and the translation of their mRNAs in an appropriate host. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of fungal cell expression vectors may be used to express HPV6a or fragments thereof in fungal cells. Commercially available fungal cell expression vectors which may be suitable for recombinant HPV6a expression include but are not limited to pYES2 (Invitrogen), *Pichia* expression vector (Invitrogen).

The expression vector may be introduced into host cells via any one of a number of techniques including but not limited to transformation, transfection, lipofection, protoplast fusion, and electroporation. The expression vector-containing cells are clonally propagated and individually analyzed to determine whether they produce HPV6a protein. Identification of HPV6a expressing host cell clones may be done by several means, including but not limited to immunological reactivity with anti-HPV6a antibodies, and the presence of host cell-associated HPV6a activity, such as HPV6a-specific ligand binding or signal transduction defined as a response mediated by the interaction of HPV6a-specific ligands at the HPV6a.

Expression of HPV DNA fragments may also be performed using *in vitro* produced synthetic mRNA or native mRNA. Synthetic mRNA or mRNA isolated from HPV6a producing cells can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

Following expression of HPV6a proteins in a host cell, HPV6a protein is be recovered to provide HPV6a in purified form.

It is apparent to those skilled in the art that the methods for producing monospecific antibodies may be utilized to produce antibodies specific for HPV polypeptide fragments, or full-length nascent HPV polypeptides. Specifically, it is apparent to those skilled in the art that monospecific antibodies may be generated which are specific for the fully functional HPV proteins or fragments thereof.

Methods for screening for compounds which modulate the expression of DNA or RNA encoding HPV as well as the function(s) of HPV6a protein(s) *in vivo* are disclosed. Compounds which modulate these activities may be DNA, RNA, peptides, proteins, or non-proteinaceous organic molecules. Compounds may modulate by increasing or attenuating the expression of DNA or RNA encoding HPV6a, or the function of HPV6a protein. Compounds that modulate the expression of DNA or RNA encoding HPV6a or the function of HPV6a protein may be detected by a variety of assays. The assay may be a simple "yes/no" assay to determine whether there is a change in expression or function. The assay may be made quantitative by comparing the expression or function of a test sample with the levels of expression or function in a standard sample.

Kits containing HPV6a DNA, fragments of HPV6a DNA, antibodies to HPV6a DNA or HPV6a protein, HPV6a RNA or HPV6a protein may be prepared. Such kits are used to detect DNA which hybridizes to HPV6a DNA or to detect the presence of HPV6a protein(s) or peptide fragments in a sample. Such characterization is useful for a variety of purposes including but not limited to forensic analyses and epidemiological studies.

Nucleotide sequences that are complementary to the HPV6a encoding DNA sequence may be synthesized for antisense therapy. These antisense molecules may be DNA, stable derivatives of DNA such as phosphorothioates or methylphosphonates, RNA, stable derivatives of RNA such as 2'-*O*-alkylRNA, or other HPV6a antisense oligonucleotide mimetics. HPV6a antisense molecules may be introduced into cells by microinjection, liposome encapsulation or by expression from vectors harboring the antisense sequence. HPV6a antisense therapy may be particularly useful for the treatment of diseases where it is beneficial to reduce HPV6a activity.

The term "chemical derivative" describes a molecule that contains additional chemical moieties which are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.

Compounds identified according to the methods disclosed herein may be used alone at appropriate dosages defined by routine testing in order to obtain optimal inhibition of the HPV6a or its activity while minimizing any potential toxicity. In addition, co-administration or sequential administration of other agents may be desirable.

Advantageously, compounds of the present invention may be administered in a single daily dose, or the total daily dosage may be administered in several divided doses. Furthermore, compounds for the present invention may be administered via a variety of routes including but not limited to intranasally, transdermally, by suppository, orally, and the like.

For combination treatment with more than one active agent, where the active agents are in separate dosage formulations, the active agents can be administered concurrently, or they each can be administered at separately staggered times.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound thereof employed. A physician of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

In the methods of the present invention, the compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrup, suppositories, gels and the like, and consistent with conventional pharmaceutical practices.

For instance, for oral administration in the form of a tablet or capsule, the active drug component can be combined with an oral, nontoxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

For liquid forms the active drug component can be combined in suitably flavored suspending or dispersing agents such as the synthetic and natural gums, for example, tragacanth, acacia, methylcellulose and the like. Other dispersing agents which may be employed include glycerin and the like. For parenteral administration, sterile suspensions and solutions are desired. Isotonic preparations which generally contain suitable preservatives are employed when intravenous administration is desired.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinyl-pyrrolidone, pyran copolymer, polyhydroxypropylmethacryl-amidephenol, polyhydroxyethylaspartamidephenol, or polyethyl-eneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

The following examples illustrate the present invention without, however, limiting the same thereto.

### EXAMPLE 1

### Preparation of Yeast Strain U9

Saccharomyces cerevisiae strain 2150-2-3 (*MATalpha, leu2-04, ade1,* cir°) was obtained from Dr. Leland Hartwell (University of Washington, Seattle, WA). Cells of strain 2150-2-3 were propagated overnight at 30°C in 5 mL of YEHD medium (Carty et al., J. Ind Micro 2 (1987)117-121). The cells were washed 3 times in sterile, distilled water, resuspended in 2 mL of sterile distilled water, and 0.1 mL of cell suspension was plated onto each of six 5-fluoro-orotic acid (FOA) plates in order to select for *ura3* mutants (Cold Spring Harbor Laboratory Manual for Yeast Genetics). The plates were incubated at 30°C. The medium contained per 250 mL distilled water: 3.5 g, Difco Yeast Nitrogen Base without amino acids and ammonium sulfate; 0.5 g 5-Fluoro-orotic acid; 25 mg Uracil; and 10.0 g Dextrose.

The medium was sterilized by filtration through 0.2 µm membranes and then mixed with 250 mL of 4% Bacto-Agar (Difco) maintained at 50°C, 10 mL of a 1.2 mg/mL solution of adenine, and 5 mL of L-leucine solution (180 mg/50 mL). The resulting medium was dispensed at 20 mL per petri dish.

After 5 days of incubation, numerous colonies had appeared. Single colonies were isolated by restreaking colonies from the initial FOA plates onto fresh FOA plates which were then incubated at 30°C. A number of colonies from the second set of FOA plates were tested for the presence of the *ura3* mutation by replica-plating onto both YEHD plates and uracil-minus plates. The desired result was good growth on YEHD and no growth on uracil-minus medium. One isolate (U9) was obtained which showed these properties. It was stored as a frozen glycerol stock (strain #325) at -70°C for later use.

### EXAMPLE 2

### Preparation of a Vector for disruption of the Yeast MNN9 gene

In order to prepare a vector for disruption of the *MNN9* gene, it was necessary to first clone the *MNN9* gene from S. cerevisiae genomic DNA. This was accomplished by standard Polymerase Chain Reaction (PCR) technology. A 5' sense primer and 3' antisense primer for PCR of the full-length *MNN9* coding sequence were designed based on the published sequence for the yeast *MNN9* gene (Zymogenetics: EPO Patent Application No. 88117834.7, Publication No. 0-314-096-A2). The following oligodeoxynucleotide primers containing flanking HindIII sites (underlined) were used:

The initiating methionine codon for the MNN9 gene is highlighted in bold print. The PCR was conducted using genomic DNA from S. cerevisiae strain JRY188 (Rine et al.) as template, Taq DNA polymerase (Perkin Elmer) and 25 cycles of amplification (94°C 1 min., 37°C 2 min., 72°C 3 min.). The resulting 1.2 kbp PCR fragment was digested with HindIII, gel-purified, and ligated with HindIII-digested, alkaline-phosphatase treated pUC13 (Pharmacia). The resulting plasmid was designated p1183.

In order to disrupt the MNN9 gene with the yeast *URA3* gene, the plasmid pBR322-URA3 (which contains the 1.1 Kbp HindIII fragment encoding the S. cerevisiae *URA3* gene subcloned into the HindIII site of pBR322) was digested with HindIII and the 1.1 kbp DNA fragment bearing the functional *URA3* gene was gel-purified, made blunt-ended with T4 DNA polymerase, and then ligated with PmlI-digested plasmid p1183 (PmlI cuts within the MNN9 coding sequence). The resulting plasmid p1199 contains a disruption of the *MNN9* gene by the functional *URA3* gene.

### EXAMPLE 3

### Construction of U9-derivative strain 1372 containing disruption of MNN9 gene

For disruption of the *MNN9* gene in strain U9 (#325), 30 µg of plasmid p1199 were digested with HindIII to create a linear *mnn9::URA3* disruption cassette. Cells of strain 325 were transformed with the HindIII-digested p1199 DNA by the spheroplast method (Hinnen et al., 1978, Proc. Natl. Acad. Sci. USA 75:1929-1933) and transformants were selected on a synthetic agar medium lacking uracil and containing 1.0 M sorbitol. The synthetic medium contained, per liter of distilled water: Agar, 20 g; Yeast nitrogen base w/o amino acids, 6.7 g; Adenine, 0.04 g; L-tyrosine, 0.05 g; Sorbitol, 182 g; Glucose, 20 g; and Leucine Minus Solution #2, 10 ml. Leucine Minus Solution #2 contains per liter of distilled water: L-arginine, 2 g; L-histidine, 1 g; L-Leucine, 6 g; L-Isoleucine, 6 g; L-lysine, 4 g; L-methionine, 1 g; L-phenylalanine, 6 g; L-threonine, 6 g; L-tryptophan, 4 g.

The plates were incubated at 30°C for five days at which time numerous colonies had appeared. Chromosomal DNA preparations were made from 10 colonies and then digested with EcoRI plus HindIII. The DNA digests were then evaluated by Southern blots (J. Sambrook et al., Molecular Cloning: A Laboratory Manual, 2nd edition, Cold Spring Harbor Laboratory Press, 1989) using the 1.2 kbp HindIII fragment bearing the *MNN9* gene (isolated from plasmid p1199) as a probe. An isolate was identified (strain #1372) which showed the expected DNA band shifts on the Southern blot as well as the extreme clumpiness typically shown by *mnn9* mutants.

### EXAMPLE 4

### Construction of a Vector for Disruption of Yeast HIS3 Gene

In order to construct a disruption cassette in which the S. cerevisiae *HIS3* gene is disrupted by the *URA3* gene, the plasmid YEp6 (K. Struhl et al., 1979, Proc. Natl. Acad. Sci., USA 76:1035) was digested with BamHI and the 1.7 kbp BamHI fragment bearing the *HIS3* gene was gel-purified, made blunt-ended with T4 DNA polymerase, and ligated with pUC18 which had been previously digested with BamHI and treated with T4 DNA polymerase. The resulting plasmid (designated p1501 or pUC18-HIS3) was digested with NheI (which cuts in the *HIS3* coding sequence), and the vector fragment was gel-purified, made blunt-ended with T4 DNA polymerase, and then treated with calf intestine alkaline phosphatase. The *URA3* gene was isolated from the plasmid pBR322-URA3 by digestion with HindIII and the 1.1 kbp fragment bearing the *URA3* gene was gel-purified, made blunt-ended with T4 DNA polymerase, and ligated with the above pUC18-HIS3 NheI fragment. The resulting plasmid (designated pUC18-his3::URA3 or p1505) contains a disruption cassette in which the yeast *HIS3* gene is disrupted by the functional *URA3* gene.

### EXAMPLE 5

### Construction of Vector for Disruption of Yeast PRB1 Gene by the HIS3 Gene

Plasmid FP8ΔH bearing the S. cerevisiae *PRB1* gene was provided by Dr. E. Jones of Carnegie-Mellon Univ. (C. M. Moehle et al., 1987, Genetics 115:255-263). It was digested with HindIII plus XhoI and the 3.2 kbp DNA fragment bearing the *PRB1* gene was gel-purified and made blunt-ended by treatment with T4 DNA polymerase. The plasmid pUC18 was digested with BamHI, gel-purified and made blunt-ended by treatment with T4 DNA polymerase. The resulting vector fragment was ligated with the above *PRB1* gene fragment to yield the plasmid pUC18-PRB1. Plasmid YEp6, which contains the *HIS3* gene, was digested with BamHI. The resulting 1.7 kbp BamHI fragment bearing the functional *HIS3* gene was gel-purified and then made blunt-ended by treatment with T4 DNA polymerase. Plasmid pUC18-PRB1 was digested with EcoRV plus NcoI which cut within the *PRB1* coding sequence and removes the protease B active site and flanking sequence. The 5.7 kbp EcoRV-NcoI fragment bearing the residual 5' and 3'-portions of the *PRB1* coding sequence in pUC18 was gel-purified, made blunt-ended by treatment with T4 DNA polymerase, dephosphorylated with calf intestine alkaline phosphatase, and ligated with the blunt-ended *HIS3* fragment described above. The resulting plasmid (designated pUC18-prb1::HIS3, stock #1245) contains the functional *HIS3* gene in place of the portion of the *PRB1* gene which had been deleted above.

### EXAMPLE 6

### Construction of a U9-related Yeast Strain containing disruptions of both the MNN9 and PRB1 Genes

The U9-related strain 1372 which contains a *MNN9* gene disruption was described in Example 3. Clonal isolates of strain 1372 were passaged on FOA plates (as described in Example 1) to select *ura3* mutants. A number of *ura3* isolates of strain 1372 were obtained and one particular isolate (strain 12930-190-S1-1) was selected for subsequent disruption of the *HIS3* gene. The pUC18-his3::URA3 gene disruption vector (p1505) was digested with XbaI plus EcoRI to generate a linear *his3::URA3* disruption cassette and used for transformation of strain 12930-190-S 1-1 by the lithium acetate method (Methods in Enzymology, 194:290 (1991). Ura⁺ transformants were selected on synthetic agar medium lacking uracil, restreaked for clonal isolates on the same medium, and then replica-plated onto medium lacking either uracil or histidine to screen for those isolates that were both Ura⁺ and His⁻. One isolate (strain 12930-230-1) was selected for subsequent disruption of the *PRB1* gene. The *PRB1* gene disruption vector (pUC18-prb1::HIS3, stock #1245) was digested with SacI plus XbaI to generate a linear *prb1::HIS3* disruption cassette and used for transformation of strain 12930-230-1 by the lithium acetate method. His⁺ transformants were selected on agar medium lacking histidine and restreaked on the same medium for clonal isolates. Genomic DNA was prepared from a number of the resulting His⁺ isolates, digested with EcoRI, and then electrophoresed on 0.8% agarose gels. Southern blot analyses were then performed using a radio-labeled 617 bp probe for the *PRB1* gene which had been prepared by PCR using the following oligodeoxynucleotide primers:

Eleven isolates were obtained which showed the expected hybridization of the probe with a 2.44 kbp *prb1 ::HIS3* DNA fragment. This was in contrast to hybridization of the probe with the 1.59 kbp fragment for the wild-type *PRB1* gene. One of these isolates containing the desired *prb1 ::HIS3* disruption was selected for further use and was designated strain #1558.

### EXAMPLE 7

### Construction of a Vector for Disruption of Yeast PEP4 Gene

The S. cerevisiae *PEP4* gene was cloned from a yeast genomic library in the following manner. E. coli cells containing the yeast genomic library pLS101 (Schultz and Friesen, 1983, J. Bacteriol. 155: 8-14)) were propagated overnight in 5 mL of LB medium containing 100 µg/mL ampicillin. From this culture, 10⁻⁴ and 10⁻⁵ dilutions were plated on LB plus ampicillin plates. Colony plates lifts were prepared using nitrocellulose filters. A 600 bp probe for the yeast *PEP4* gene was prepared by PCR using Taq DNA polymerase, total plasmid DNA from the pLS101 yeast library, and the following oligodeoxynucleotide primers designed based on the published DNA sequence for *PEP4* (C. A. Woolford et al., Mol. Cell. Biol. 6:2500 (1986)). The PCR was conducted with 25 cycles of amplification (94°C 1 min., 37°C 2 min., 72°C 3 min.). The PCR probe was gel-purified, radio-labeled, and hybridized with the above colony filters. Several colonies were positive for hybridization with the *PEP4* probe and were restreaked on LB plus ampicillin plates for single colonies. Plasmid DNA was prepared by the alkaline-SDS lysis (Sambrook et al., supra) from several of the isolates and digested with BamHI. The expected 14 kbp vector band and 6.9 kbp *PEP4* insert band were observed. Upon double digestion with EcoRI plus XhoI, the expected 1.5 kbp band for *PEP4* was observed. One isolate (*E. coli* strain #860) showing the expected results was selected for further use. Plasmid DNA from strain #860 was digested with BamHI and the 6.9 kbp BamHI DNA fragment carrying the chromosomal *PEP4* gene was subcloned into the BamHI site of pUC13 to yield the plasmid p890. The plasmid p890 was then digested with NcoI (which cuts within the *PEP4* coding sequence), gel-purified, made blunt-ended by treatment with T4 DNA polymerase, and ligated with the 1.1 kbp blunt-ended fragment bearing the functional *URA3* gene (prepared as in Example 2). The resulting plasmid containing the *PEP4* gene disrupted by the *URA3* gene was designated pUC13-pep4::URA3 (strain #906).

### EXAMPLE 8

### Construction of Yeast Strains #1569, #1538 and #1592, which are derivatives of Strain U9 Containing both prb1 and pep4 Mutations

In order to disrupt the *HIS3* gene in strain U9, the disruption vector pUC18-his3::URA3 was digested with EcoRI plus XbaI and then used to transform strain U9 by the lithium acetate method. Ura⁺ transformants were selected on agar medium lacking uracil and restreaked on the same medium for clonal isolates. A number of the resulting Ura⁺ isolates were then replica-plated onto agar medium lacking either uracil or histidine and screened for those that were both Ura⁺ and His⁻. One isolate (strain #1524) was selected for subsequent disruption of the *PRB1* gene. The *PRB1* gene disruption vector pUC18-prbl ::HIS3 was digested with SacI plus XbaI and then used for transformation of strain #1524 by the lithium acetate method. His⁺ transformants were selected on agar medium lacking histidine and restreaked for clonal isolates on the same medium. Genomic DNA was prepared from a number of the His⁺ isolates and evaluated by Southern blot hybridization with a radio-labeled *PRB1* probe. One of the isolates (strain #1537) showing the desired disruption of the *PRB1* gene by *HIS3* (i.e., prb1::HIS3) was selected for subsequent disruption of the *PEP4* gene. Strain #1537 was passaged on FOA plates in order to obtain *ura3* isolates and one isolate (strain #1541) was selected for further use.

In order to disrupt the *PEP4* gene in strain #1541, the *PEP4* gene disruption vector pUC13-*pep4::URA3* was digested with XhoI to generate a linear *pep4::URA3* disruption cassette and used for transformation of strain #1541 by the lithium acetate method. Ura⁺ transformants were selected on uracil-minus agar medium and streaked for clonal isolates on the same medium. Genomic DNA was prepared from a number of the Ura⁺ transformants and evaluated by Southern blots using a radio-labeled probe for the *PEP4* gene. One isolate (strain #1569) showing the desired disruption of the *PEP4* gene by the *URA3* gene was selected for further use. In an independent experiment following virtually the same sequence of steps, strain #1538 was constructed. Strain #1538 is a derivative of strain U9. Strain #1538 contains both *prb1* and *pep4* mutations. In addition, a *ura3* derivative of strain #1569 was obtained by passage on FOA plates. The resulting *ura3* derivative of strain #1569 was designated as strain #1592.

### EXAMPLE 9

### Extraction of nucleic acid from biopsy.

A large vulvar condyloma acuminatum lesion was obtained from a 25 year old, post partum female patient. A fragment of the lesion was frozen in liquid nitrogen, then processed with a Braun mikro-dismembrator II (B. Braun Instruments, Melsungen , Germany). The resulting material was solubilized with 0.6% (w/v) sodium dodecyl sulfate (SDS), treated with proteinase K (50 mcg/ml), and extracted with phenol/chloroform/isoamyl alcohol. DNA was ethanol-precipitated and quantified by UV spectrophotometry. The presence of high-molecular-weight DNA was established by agarose gel electrophoresis followed by staining with ethidium bromide.

### EXAMPLE 10

### Typing of HPV DNA

The HPV DNA type was determined using the hybrid capture assay marketed as ViraType Plus (Digene Diagnostics, Beltsville, MD). The HPV probes used were divided into two pools whose composition is based on the association of each type with genital tract malignancies. Probe group A contained the "low-risk" types HPV6, 11, 42, 43, and 44 while probe B contained the "high-risk" types 16, 18, 31, 33, 35, 45, 51, 52, and 56. Total DNA was digested with PstI, BamHI, and HindIII and Southern blots were performed under high stringency conditions (Tₘ-15°C) to determine the HPV subtype.

### EXAMPLE 11

### Cloning of HPV6a genome

Total DNA extracted from the HPV6a-positive biopsy sample was digested with HindIII endonuclease. Following size-fractionation through a 0.8% low-melting-temperature agarose preparative gel, a region corresponding to DNA of ∼ 8 kbp was excised from the gel and the agarose was digested with Gelase™ enzyme (Epicentre Technologies, Inc., Madison, WI). The sample was ligated with pUC18 (Pharmacia, Inc., Piscataway, NJ) which had been digested with HindIII and dephosphorylated . Following transformation of competent *E. coli* DH5 cells (Gibco, BRL, Gaithersburg, MD), the plasmid library was screened for HPV6a-positive clones by colony-hybridization using an antisense ³²P-labeled oligodeoxynucleotide that was complementary to the 3'-end of the HPV6b L1 gene (5'-GAG AGA TCT TAC CTT TTA GTT TTG GCG CGC TTA C-3'). A pUC18 plasmid containing the 8.1-kbp HPV6a genome was isolated and characterized by restriction enzyme and Southern blot analyses. This plasmid was designated pUC18-HPV6a. Plasmid DNA was prepared using the Qiagen™ Plasmid Maxi kit (Qiagen Inc., Chatsworth, CA).

### EXAMPLE 12

### Sequence analysis of pUC18-HPV6a.

To determine the complete HPV6a sequence, sequencing primers were synthesized based on the published HPV6b sequence. Both strands of the complete 8.1-kbp HPV6a genome were sequenced by the dideoxy chain termination method using the PRISM™ kit and an Applied Biosystems (ABI) automated sequencer (#373A) according to the manufacturers' instructions (ABI, Inc., Foster City, CA). In cases where the sense and antisense sequence did not match, additional HPV6a specific primers were synthesized to resequence in both directions over the area in question to obtain a consensus.

The DNA sequences of HPV6a and HPV6b exhibited over 97% identity with a total of 229 bp changes identified out of 8010 bp. The most significant differences compared to the HPV6b sequence were found in the long control region (LCR; nt 7205 - nt 106). Apart from several single nucleotide (nt) changes in the HPV6a LCR, a 94-bp insertion at nt 7350 and another 19-bp insertion at nt 7804 were found. At nt 7615, six base pairs were deleted from the HPV6a genome.

### EXAMPLE 13

### Construction of HPV6a L1 Yeast Expression Vector

HPV type 6a DNA was used as a template for PCR. The HPV6a L1 gene was amplified by PCR using Vent polymerase (New England Biolabs, Inc.), 35 cycles of amplification (9.4°C 1 min, 48°C 1 min, 72°C 1 min 45 sec), and the following oligodeoxynucleotide primers which contain flanking Bgl II sites (underlined):

The sense primer introduces a yeast non-translated leader sequence immediately upstream to the HPV6a L1 initiating methionine codon (highlighted in bold print). The 1.5-kbP L1 PCR product was digested with BglII and gel-purified. The pGAL10 expression vector was constructed by isolating a 1.4 kbp SphI fragment from a bidirectional GAL promoter vector pUC18-GAL1p-GAL10p which contains the divergent *GAL1*/*GAL10* promoter from the plasmid pBM272 (provided by Dr. Mark Johnston, Washington University, St. Louis) flanked on each side by a copy of the yeast *ADH1* transcriptional terminator [Bennetzen, J.L. and Hall, B.D. (1982) J. Biol. Chem. 257:3018-3025], cloning a BamHI site located between the *GAL1* promoter and the first copy of the *ADH1* transcriptional terminator, and a SmaI cloning site located between the divergent *GAL10* promoter and the second copy of the *ADH1* transcriptional terminator. A yeast shuttle vector consisting of pBR322, the yeast LEU2-d gene and the yeast 2 µm circle with SphI and ligated with the 1.4-kbp SphI *GAL* promoter fragment. The resulting vector, pGAL10, was linearized with BamHI which cuts between the *GAL1* promoter and the *ADH1* transcription terminator. The BamHI-digested pGAL10 vector and the BglII digested HPV6a L1 PCR fragment were ligated and used to transform *E. coli* DH5 cells (BRL). A pC1/1-GAL plasmid was isolated which contains the HPV-6a L1 gene and was designated p13173-357-6. The L1 gene in p13173-357-6 was sequenced (ABI Sequencer #373A) and shown to be identical to the L1 gene in the pUC18-HPV6a clone.

### EXAMPLE 14

### Construction of the HPV6a L1 and L2 Yeast Expression Vector

Plasmid p13173-357-6 (pGAL10 + HPV6a L1) was digested with Smal, which cuts between the *GAL10* promoter and the *ADH1* transcription terminator. The 1.4-kbp HPV6a L2 gene was amplified by PCR using the pUC18-HPV6a DNA as template, Vent polymerase (New England Biolabs, Inc.), 10 cycles of PCR amplification (94°C, 1 min; 48°C, 1 min; 72°C, 1 min 45 sec) and the following oligodeoxynucleotide primers which contain flanking SmaI sites (underlined):

The sense primer introduces a yeast non-translated leader sequence immediately upstream to the HPV6a L2 initiating methionine codon (highlighted in bold print). The PCR fragment was digested with SmaI, gel purified and ligated with the Smal digested p13173-357-6 plasmid. A pGAL10 plasmid containing both the HPV6a L1 and L2 genes was isolated and designated, p14049-7-2. The L2 gene was sequenced (ABI Sequencer #373A) and found to be identical to the L2 gene in the pUC18-HPV6a clone.

### EXAMPLE 15

### Expression of HPV6a L1 and Co-Expression of HPV6a L1 and L2 in Yeast

Plasmids p13173-357-6 (pGAL10 + HPV6a L1) and p14049-7-2 (pGAL10 + HPV6a L1 and L2) were used to transform S. cerevisiae strains #1569 (*MATa, leu2-04, prb1, ade1, pep4,* cir°) and #1558 (*MATa, leu2-04, prb1, mnn9, ade1*, cir°). The resulting recombinant strains obtained using host strain #1558 were strains #1644 (HPV6a L1) and #1670 (HPV6a L1+L2) as shown in the table. Clonal isolates were grown at 30°C in YEHD medium containing 2% galactose for 68-78 hours. After harvesting the cells, the cell pellets were broken with glass beads and cell lysates analyzed for the expression of HPV6a L1 or HPV6a L2 protein by immunoblot analysis. Samples containing 40 µg of total cellular protein were electrophoresed on 10% Tris-Glycine gels under reducing and denaturing conditions and electroblotted onto nitrocellulose filters. The L1 protein was immunodetected using rabbit antisera raised against a trpE-HPV11 fusion protein (Brown, D. R. et al., Virology 201:46-54) as primary antibody and donkey anti-rabbit IgG horseradish peroxidase-linked (HRP) whole antibody (Amersham, Inc.) as the secondary antibody. The filters were processed using the chemiluminescent ECL™ Detection Kit (Amersham, Inc.). A 50-55 kDa L1 protein band was detected in all samples except the negative control (vector control without L1 or L2 gene).

The L2 protein was detected as a 70 kDa protein band by Western analysis using 1:250 diluted sera from mice which had been immunized 3 times with a trpE-HPV6a L2 fusion protein prepared essentially according to the method of Carter et al. as the primary antibody and HRP-linked, sheep anti-mouse IgG (Amersham, Inc.) as a second antibody (1:1000 dilution).

For EM analysis (Structure Probe, West Chester, PA), an aliquot of each sample was placed on 200 mesh carbon coated copper grids. A drop of 2% phosphotungstic acid (PTA), pH 7.0 was placed on the grid for 20 seconds. The grids were allowed to air dry prior to TEM examination. All microscopy was done using a JEOL 100CX transmission electron microscope (JEOL USA, Inc.) at an accelerating voltage of 100 KV. The micrographs generated have a final magnification of 100,000X.

VLP were observed in the 50-55 nm diameter size range in all yeast samples harboring either the HPV6a L1 or HPV6a L1 and L2 coexpression plasmids. No VLP were observed in yeast control samples.

### EXAMPLE 16

### Fermentation of HPV6a L1+L2 (Strain #1670).

Surface growth of a plate culture of strain 1670 was aseptically transferred to a leucine-free liquid medium containing (per L): 8.5 g Difco yeast nitrogen base without amino acids and ammonium sulfate; 0.2 g adenine; 0.2 g uracil; 10 g succinic acid; 5 g ammonium sulfate; and 0.25 g L tyrosine; this medium was adjusted to pH 5.0-5.3 with NaOH prior to sterilization. After growth for 25 hr at 28 °C, 250 rpm on a rotary shaker, frozen culture vials were prepared by adding sterile glycerol to a final concentration of 17% (w/v) prior to storage at -70 C (1 mL per cryovial). Inoculum for fermentation of strain 1670 was developed in the same medium (500 mL per 2-L flask) and was started by transferring the thawed contents of two frozen culture vials to the 2-L flasks and incubating at 28 °C. 250 rpm on a rotary shaker for 25 hr. Fermentation of strain 1670 used a New Brunswick SF-116 fermentor with a working volume of 10 L after inoculation. The production medium used contained (per L): 20 g Difco yeast extract; 10 g Sheffield HySoy peptone; 20 g glucose; 20 g galactose; the medium was adjusted to pH 5.3 prior to sterilization. The entire contents (500 mL) of the 2-L inoculum flask was transferred to the fermentor which was incubated at 28 °C, 5 L air per min, 400 rpm, 3.5 psi pressure. Agitation was increased as needed to maintain dissolved oxygen levels of greater than 40% of saturation. Progress of the fermentation was monitored by offline glucose measurements (Beckman Glucose 2 Analyzer) and online mass spectrometry (Perkin-Elmer 1200). After 69 hr incubation, a cell density of 9.9 g dry cell weight per L was reached. The culture was concentrated by hollow fiber filtration (Amicon H5MP01-43 cartridge in an Amicon DC-10 filtration system) to ca. 2 L, diafiltered with 2 L phosphate-buffered saline, and concentrated further (to ca. 1 L) before dispensing into 500 mL centrifuge bottles. Cell pellets were collected by centrifugation at 8,000 rpm (Sorval GS3 rotor) for 20 min at 4 °C. After decanting the supernatant, the pellets (total 225 g wet cells) were stored at - 70 °C until use.

### EXAMPLE 17 (Reference)

### Purification of Recombinant HPV6a L1 + L2 Capsid Proteins

All steps performed at 4 ° C unless noted.

Cells of strain #1670 were stored frozen at -70 ° C. Frozen cells (wet weight = 38.0 g) were thawed at 20-23 ° C and resuspended in 50 mL "L1 Buffer" (20 mM sodium phosphate, pH 7.2, 100 mM NaCl, 1.7 mM EDTA). The protease inhibitors PMSF and pepstatin A were added to final concentrations of 2 mM and 1.7 mcM, respectively. The cell slurry was broken at a pressure of approximately 8,000 psi by 3 passes in a M110 Microfluidizer (Microfluidics Corp., Newton, MA). The broken cell slurry was centrifuged at 5,000 x g for 10 min to remove cellular debris. The supernatant liquid containing L1 + L2 antigen was recovered.

The supernatant liquid was diluted 1:5 by addition of Buffer A (20 mM MOPS, pH 7.0) and applied to an anion exchange capture column (5.0 cm ID x 4.0 cm) of Fractogel ® EMD TMAE-650 (S) resin (EM Separations, Gibbstown, NJ) equilibrated in Buffer A. Following a wash with Buffer A, the antigen was eluted with a gradient of 0 - 1.0 M NaCI in Buffer A. Immuno-dot blotting was performed to determine which fractions from the column contained L1 protein.

Fractions containing L1 protein as determined by immuno-dot blot were assayed for total protein by the Bradford method followed by SDS-PAGE using silver staining and Western blotting.

TMAE fractions showing comparable purity and enrichment of L1 protein were pooled. The antigen was concentrated by ammonium sulfate fractionation. The solution was adjusted to 63% saturated ammonium sulfate by adding solid reagent while gently stirring over 30 min. The sample was placed on ice and precipitation allowed to proceed for 30 min. The sample was centrifuged at 12,000 x g. The pellet was resuspended in 20.0 mL PBS (6.25 mM Na phosphate, pH 7.2, 150 mM NaCl).

The resuspended pellet was chromatographed on a size exclusion column (2.6 cm ID x 89 cm) of Sephacryl 500 HR resin (Pharmacia, Piscataway, NJ). Running buffer was PBS. Chromatography performed at 20-23 ° C. Fractions were analyzed by SDS-PAGE using silver staining and western blot detection. The purest fractions were pooled. The resulting pool was concentrated.

Final product was analyzed by SDS-PAGE using colloidal Coomassie staining. The L1 and L2 proteins were estimated to be 85% homogeneous. The identity of L1 and L2 proteins was confirmed by western blotting using the appropriate antisera. The final product was aliquoted and stored at -70 ° C. This process resulted in a total of 3.0 mg protein.

Electron microscopy analysis was performed by Structure Probe (West Chester, PA). An aliquot of sample was placed on a 200 mesh carbon-coated copper grid. A drop of 2% phosphotungstic acid, pH 7.0 was placed on the grid for 20 seconds. The grid was allowed to air dry prior to TEM examination. All microscopy was performed using a JEOL 100 CX transmission electron microscope (JEOL USA, Inc.) at an accelerating voltage of 100 kV. The micrographs generated have a final magnification of 100,000 x. The presence of virus-like particles in the 50-55 nm size range was confirmed.

### EXAMPLE 18

### Construction of CRPV L1 Expression Vector

The CRPV L1 gene was amplified by PCR from plasmid pLAII which contains the complete CRPV viral genome (Dr. Peter Howley, NCI) using Vent polymerase (New England Biolabs, Inc.); 35 cycles of amplification (94°C, 1 min; 50°C, 1 min; 72°C, 2 min) and the following oligonucleotide primers which contain flanking BglII sites (underlined) were used:

The sense primer introduces a yeast non-translated leader sequence immediately upstream of the CRPV L1 initiating methionine codon (highlighted in bold print). The 1.6 kb L1 PCR product was digested with BglII, gel-purified and subcloned into the Bgl II site of vector pSP72 (Promega) to yield the plasmid pSP72-CRPV-L1 (p12930-314-4-1).

One subclone was sequenced completely and found to be different in 4 nucleotides from the published CRPV L1 sequence. The changes do not result in amino acid changes. The L1 gene was cut out of pSP72-CRPV-L1 as a Bgl II fragment and subcloned into the unique BamHI site located between the yeast *GALl0* promoter and *ADH1* transcriptional terminator in a yeast expression vector which contains the *GAL10* promoter from YEp52 (Broach et al., Exp. Manipulation of Gene Expression, 1983, 83:81-116) and *ADH1* transcriptional terminator in the same vector backbone). The resulting plasmid was designated p12930-323-6-1.

### EXAMPLE 19

### Construction of CRPV L2 Expression Vector

The CRPV L2 gene was amplified by PCR using Vent polymerase (New England Biolabs, Inc.) from plasmid pLAII after digesting the plasmid with Sail, gel purifying the 7.9 Kb fragment and ligating it with itself. Thirty-five cycles of amplification (90°C, 1 min; 50°C, 1 min; 72°C, 2 min) and oligonucleotide primers which contain flanking EcoRI sites (underlined) were used:

The sense primer introduces a yeast non-translated leader sequence immediately upstream of the CRPV L2 initiating methionine codon (highlighted in bold print). The 1.5 kb PCR product was digested with EcoRI, gel-purified and subcloned into the EcoRI site of the bidirectional promoter vector pUC18-GAL1p-GAL10p containing the divergent yeast *GAL1*/*GAL10* promoter. This vector contains a unique BamHI site between the *GAL1* promoter and a first copy of the *ADH1* transcription terminator, unique EcoRI and SmaI sites located between the *GAL10* promoter, and a second copy of the *ADH1* transcriptional terminator. The resulting expression cassette is carried on a 1.4 kb SphI fragment. One clone (p12930-295-2-2) containing the desired L2 insert adjacent to the *GAL10* promoter was completely sequenced and shown to contain 6 nucleotide changes from the published sequence, 4 of which result in amino acid changes. Sequence analysis of the original template DNA confirmed that the changes were also present in pLAII and not introduced by the PCR. The pUC18-GAL1p-GAL10p vector containing the L2 gene was cut with SphI and the 2.9 kb fragment harboring the ADH1t-GAL1p-GAL10p-L2-ADH1t expression cassette was ligated with the large Sph1 fragment of the yeast shuttle vector. The resulting plasmid was designated p12930-323-2-3.

### EXAMPLE 20

### Expression of CRPV L1 and L2 Capsid Proteins in Yeast

Plasmids p12930-323-6-1 and p12930-323-2-3 were used to transform S. cerevisiae strains #1569, #1538, BJ5462 [E. W. Jones, Methods in Enzymology 194 (1991) 428-4531 and BJ1995 [Jones, ibid.]. Clonal isolates were grown at 30°C in YEHD medium containing 2% galactose for 48-72 hours. After harvesting the cells, the cell pellets were broken with glass beads, TritonX-100 was added to 0.5% final concentration and the resulting cell lysates were evaluated for the expression of CRPV L1 and L2 by immunoblot analyses. Samples containing 40 µg of total cellular protein were electrophoresed on 12% Tris-Glycine gels (Novex) under reducing and denaturing conditions and electroblotted onto PVDF membranes (Novex). CRPV L1 and L2 proteins were detected using polyclonal rabbit anti-L1 or anti-L2 antisera (gift of Dr. John Kreider, Hershey Medical Center) as primary antibodies and protein A coupled to horseradish peroxidase (Amersham, Inc.) as the secondary antibody. The membranes were processed using the chemiluminescent ECL™ Detection Kit (Amersham, Inc.). A 55-61 kDa L1 protein band was detected in all samples harboring the L1 expression plasmid and a ∼90 kDa L2 protein band was detected in all samples from yeast clones harboring the L2 expression plasmid.

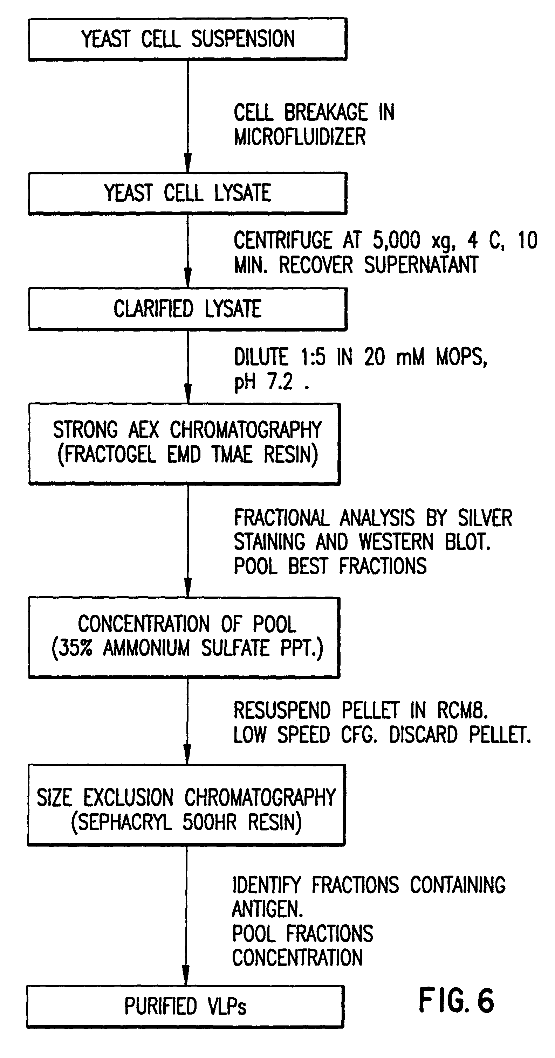

No signal was detected in samples derived from yeast clones harboring the L1 expression plasmid with anti-L2 antisera or vice versa. (Figure 6).

Based on these expression results, the following recombinant yeast strains were selected for further studies: strain #1582, which is host strain #1569 containing plasmid p12930-323-6-1; and strain #1583, which is host strain #1538 containing plasmid p12930-323-2-3.

### EXAMPLE 21 (Reference)

### A. Purification of Recombinant CRPV L1 Capsid Protein

Cells of strain #1582, stored at -70°C, were thawed and suspended in an equal volume of Breaking buffer (20 mM sodium phosphate, pH 7.2, 100 mM NaCl, 1.7 mM EDTA). Protease inhibitors PMSF and Pepstatin A were added to the slurry to final concentrations of 2 mM and 1.7 µM respectively. Cells were lysed by 10 passes in a microfluidizer. The lysate was clarified by centrifugation at 5000 x g for 10 minutes. The supematant was layered on top of a 5 cm cushion of 45% sucrose (w/v) in L1 buffer, and L1 was pelieted by centrifugation at 100,000 x g for 4 hours. The pellet was resuspended in 1/10th volume of L1 buffer. The resuspended pellet was clarified by centrifugation at 5000 x g for 10 minutes. Tween-80 was added to the supernatant to a final concentration of 0.01 % and the supernatant was fractionated at room temperature by size-exclusion chromatography on a 1700 ml column (5 cm ID) of Sephacryl S-1000 resin (Pharmacia). Running buffer for this column was 10 mM sodium phosphate, pH 7.2, 150 mM NaCl, 0.01% Tween-80. Fractions containing immunoreactive material by immunodot blot assay were pooled and concentrated to 1/6th volume by ultrafiltration using an Amicon stirred cell with a 76 mm diameter YM-100 flat-sheet membrane (100,000 MWCO). The product was sterile filtered through a Millex-GV 0.22 µm membrane (Millipore).

### B. Characterization of Recombinant CRPV L1 Capsid Protein

The identity of the final product was confirmed by Western blotting and by N-terminal sequence analysis. Purity was assessed by SDS/PAGE with Coomassie and silver staining, and by Solution Sieving Capillary Electrophoresis (SSCE) with optical detection at 215 nm. L1 was 75% pure by SSCE. Electron microscopy showed that VLP in the 50 - 55 nm diameter size range were present.

### C. Analytical Size-Exclusion HPLC

To assay for VLP's, samples were fractionated according to size by size-exclusion chromatography. Chromatography was performed with a Perkin-Elmer Series 410 Biopump HPLC with an ISS 100 autoinjector fitted with a 200 microliter injection loop. The column was a TSK-gel G5000PW, 7.5 x 600 mm (TOSOHAAS, Montgomeryville, PA). To monitor elution of protein from the column, optical detection at 280 nm was accomplished with a Perkin-Elmer LC-235 diode array detector. The mobile phase was 0.5 M NaCl in 10 mM sodium phosphate buffer, pH 7.2. The flow rate was 0.5 mL/min, and one milliliter fractions were collected. Column calibration was performed with protein standards from Sigma as well as recombinant hepatitis B surface antigen (Recombivax™, Merck & Co., West Point, PA). Detection of antigen in fractions collected during elution is accomplished by immunodot blot assay.

### D. Immunodot Blot Assay

A 10 microliter sample of each fraction was applied to a strip of PVDF membrane (Immobilon-P, Millipore Corp., Bedford, MA) which was pre-wetted and placed on dampened blotting paper. The sample was allowed to soak into the membrane, and the membrane was placed in a Blocking Solution (5% (w/v) of non-fat dry milk dissolved in 0.15 M NaCl, 0.02% (w/v) sodium azide, and 0.01M sodium phosphate, pH 7.2) and incubated at room temperature with gentle agitation for a minimum of three hours. Blocking solution was decanted and replaced with primary antibody solution.

Primary antibody used varied depending on the antigen to be detected:
CRPV L1 was probed with rabbit anti-CRPV serum "late response" (Biodesign International, Kennebunk, ME). CRPV L2 was probed with rabbit anti-CRPV L2 serum. HPV6a L1 was probed with monoclonal antibody MAB 837 (Chemicon International, Inc., Temecula, CA). HPV6a L2 was probed with mouse anti-HPV6a L2-trpE fusion serum.
Visualization of the immune complexes was by standard methods using a second antibody conjugated to alkaline phosphatase and the chromogenic substrate NBT/BCIP.

### E. Preparation of Vaccine from Recombinant CRPV L1 Capsid Protein

Purified CRPV L1 Capsid Protein was adsorbed to Al(OH)₃ at a concentration of 100 µg/ml.

### EXAMPLE 22 (Reference)

### Purification of Recombinant CRPV L1 Capsid Protein - Scheme 2

All steps were performed at 4°C unless specified.

Cells of strain #1582, stored at -70°C, were thawed and suspended in an equal volume of Breaking buffer (20 mM sodium phosphate, pH 7.2, 100 mM NaCl, 1.7 mM EDTA). Protease inhibitors PMSF and Pepstatin A were added to the slurry to final concentrations of 2 mM and 1.7 µM respectively. Cells were lysed using a BioNeb Cell Disruptor system (Glas-Col Apparatus Co., Terra Haute, IN). The lysate was clarified by centrifugation at 5000 x g for 10 minutes.

The supernatant was layered on top of a 5 cm cushion of 45% sucrose (w/v) in L1 buffer, and L1 was pelleted by centrifugation at 100,000 x g for 4 hours. The pellet was resuspended in 1/10th volume of L1 buffer. The resuspended pellet was clarified by centrifugation at 5000 x g for 10 minutes.

Supernatant was diluted 1:5 with PBS, reclarified by centrifugation in Sorvall SA-600 rotor at 6500 rpm for 10 minutes at 4°C. The supernatant was filtered through a 0.22 micron syringe filter and fractionated by anion-exchange chromatography.

Anion-exchange chromatography was performed with a Perkin-Elmer Series 410 Biopump HPLC with a 5 milliliter injection loop. The chromatography medium was a Fractogel EMF TMAE-650 (S) 25-40 micron resin (EM Separations, Gibbstown, NJ) in a 150 mm x 10 mm ID glass column. To monitor elution of protein from the column, optical detection at 280 nm was accomplished with a Perkin-Elmer LC-235 diode array detector. The column was pre-equilibrated with 0.15M NaCl in 0.01M sodium phosphate buffer , pH 7.2 (Buffer A). The column was run at a flow rate of 0.75 ml/min. The sample was loaded on the column, and the column was washed with Buffer A to remove unbound material. Bound material was eluted with a linear concentration gradient of sodium chloride, 0.15 M to 0.65 M for 5 minutes, followed by a 0.65 M to 1.15 M linear gradient for 30 minutes. Detection of antigen in fractions collected during elution was accomplished by immuno dot blot assay. Fractions containing immunoreactive material (i.e., fractions eluting between 0.81M and 1.05M NaCl) were pooled.

The pooled fractions were concentrated in Macrosep centrifugal concentration devices (Filtron Technology Corp., Northborough, MA) to 1/5th volume.

The concentrate was fractionated by size-exclusion chromatography using Sephacryl S-1000 SF resin (Pharmacia, Piscataway, NJ) in a 87 cm x 27 mm ID column. The column was run at a flow rate of 2.5 ml/min. Elution of protein was monitored by A280nm. Antigen was detected by immunoblot.

Fractions containing immunoreactive material were pooled and concentrated by ultrafiltration using a stirred cell (Amicon, Inc., Beverly, MA) with a 43 mm diameter YM-100 flat sheet membrane (Amicon, Inc., Beverly, MA) under nitrogen at 10 psi pressure.

The final product was characterized by SDS/PAGE with Coomassie staining, and by solution-sieving capillary electrophoresis (SSCE). The final product from Scheme 2 was 88% pure by SSCE.

### EXAMPLE 23 (Reference)

### A. Purification of Recombinant CRPV L1 Capsid Protein - Scheme 3 (Figure 17)

All steps were performed at 4°C unless specified.

Cells of strain #1582, stored at -70°C, were thawed and suspended in an equal volume of Breaking buffer (20 mM sodium phosphate, pH 7.2, 100 mM NaCl, 1.7 mM EDTA). Protease inhibitors PMSF and Pepstatin A were added to the slurry to final concentrations of 2 mM and 1.7 mcM respectively. Cells were lysed using a BioNeb Cell Disruptor system (Glas-Col Apparatus Co., Terra Haute, IN). The lysate was clarified by centrifugation at 5000 x g for 10 minutes.

The supernatant was layered on top of a 5 cm cushion of 45% sucrose (w/v) in L1 buffer, and L1 was pelleted by centrifugation at 100,000 x g for 4 hours. The pellet was resuspended in 1/10th volume of L1 buffer. The resuspended pellet was clarified by centrifugation at 5000 x g for 10 minutes.

The supernatant was extracted with 1/2 volume of chloroform. The aqueous layer was removed and clarified by centrifugation at 12,000 rpm in a Beckman microfuge for 5 minutes at room temperature.

Supernatant was diluted 1:5 with PBS, reclarified by centrifugation in Sorvall SA-600 rotor at 6500 rpm for 10 minutes at 4°C. The supernatant was filtered through a 0.22 micron syringe filter and fractionated by anion-exchange chromatography.

Anion-exchange chromatography was performed with a Perkin-Elmer Series 410 Biopump HPLC with a 5 milliliter injection loop. The chromatography medium was a Fractogel EMF TMAE-650 (S) 25-40 micron resin (EM Separations, Gibbstown, NJ) in a 150 mm x 10 mm ID glass column. To monitor elution of protein from the column, optical detection at 280 nm was accomplished with a Perkin-Elmer LC-235 diode array detector. The column was pre-equilibrated with 0.15M NaCl in 0.01M sodium phosphate buffer, pH 7.2 (Buffer A). The column was run at a flow rate of 0.75 ml/min. The sample was loaded on the column, and the column was washed with Buffer A to remove unbound material. Bound material was eluted with a linear concentration gradient of sodium chloride, 0.15 M to 0.65 M for 5 minutes, followed by a 0.65 M to 1.15 M linear gradient for 30 minutes. Detection of antigen in fractions collected during elution was accomplished by immuno dot blot assay. Fractions containing immunoreactive material (i.e., fractions eluting between 0.81M and 1.05M NaCI) were pooled.

The pooled fractions were concentrated in Macrosep centrifugal concentration devices (Filtron Technology Corp., Northborough, MA) to 1/5th volume.

The concentrate was fractionated by size-exclusion chromatography using Sephacryl S-1000 SF resin (Pharmacia, Piscataway, NJ) in a 87 cm x 27 mm ID column. The column was run at a flow rate of 2.5 ml/min. Elution of protein was monitored by A280nm. Antigen was detected by immunoblot.

Fractions containing immunoreactive material were pooled and concentrated by ultrafiltration using a stirred cell (Amicon, Inc., Beverly, MA) with a 43 mm diameter YM-100 flat sheet membrane (Amicon, Inc., Beverly, MA) under nitrogen at 10 psi pressure.

The final product was characterized by SDS/PAGE with Coomassie staining, and by solution-sieving capillary electrophoresis (SSCE). The final product from Scheme 3 was 95% pure by SSCE.

### Solution Sieving Capillary Electrophoresis (SSCE)

Samples of CRPV VLP (∼0.2 milligram/milliliter) were heated for 15 minutes at 100°C in 1% 2-mercaptoethanol and 1% (w/v) SDS. The sample was applied electrokinetically along with a reference marker, mellitic acid, to a silica fused capillary, 42 cm (22 cm to detector) x 0.05mm I.D., pre-equilibrated with 10 lumen volumes of 0.1 N NaOH, water, and ProSort sieving reagent (Applied Biosystems, Foster City, CA). A separation voltage of 300 volts/cm was applied using an Applied Biosystems Model 270A-HT CE instrument. Elution of sample was monitored by absorbance at 215 nm, and data was collected using Nelson Turbochrom 3 software.

### EXAMPLE 24

### Protection Against Papilloma Development Caused by CRPV by Vaccination with Yeast Derived CRPV L1 VLPs

5 New Zealand White rabbits were immunized intramuscularly with either 135 µg of L1 VLPs (75% pure) adsorbed to alum or 100 µg recombinant hepatitis B surface antigen (99% pure) adsorbed to aluminum hydroxide as a negative control. Animals received 2 more boosts over 8 weeks before they were challenged with CRPV 10 days after the last boost. Sera taken before immunization, at each boost, and before challenge were analyzed by an L1-specific ELISA. 96-well plates were coated with 5 µg crude lysate of yeast-derived CRPV-L1 or CRPV-L2. Protein was removed and plates were blocked in 10% dried milk (Carnation) + TTBS (20 mM TRIS pH 7.4, 500 mM NaCl, 0.1 % Tween) for 2 hrs at 4°C. After washing plates with TTBS, diluted rabbit sera were added in 1 % milk + TTBS and incubated for 2 hrs at 4°C. Plates were washed with TTBS and incubated with a 1:1000 dilution of anti-rabbit IgG-alkaline phosphatase (Kirkegaard and Perry Labs., Inc.) in 1% milk + TTBS for 2 hrs at 4°C. Plates were washed with TTBS and developed by adding p-nitrophenyl phosphate (Kirkegaard and Perry Labs., Inc.). The reaction was stopped by adding 5% EDTA. Absorbance was measured at 405 nm. Titer was considered positive if L1/L2 absorbance ratio was 2:1.

Anti-L1 antibody titers were present 4 weeks after the first injection, and they increased with each additional boost. Control animals were negative. Six weeks after CRPV challenge the vaccinated animals were wart-free in 15 out of 15 sites (1:2 diluted or 1:12 diluted virus stock) while the control animals showed wart formation in 12 out of 15 sites (1:2 diluted virus) or 9 out of 15 (1:12 diluted virus). After 15 weeks, the vaccinated animals were still wart-free.

Virus neutralization assays were performed (essentially according to method of Christensen et al., 1991, Virology 181:572-579) by mixing rabbit sera with CRPV and subsequently challenging rabbits with the treated CRPV. The standard for determining neutralizing antibody was complete virus neutralization (3/3 sites negative for wart formation). Sera from the 5 vaccinated animals and 5 control animals were analyzed. Sera collected after 1 dose of CRPV L1 VLPs contained antibodies which completely neutralized undiluted virus in 80% of rabbits (4/5). After 2 or 3 doses of CRPV L1 VLPs 100% of rabbits (5/5) had virus neutralizing antibodies. Control sera did not show any virus neutralizing activity. Depending on final titers sera of selected vaccinated animals could be diluted between 10-1000 fold and were still 100% neutralizing.

### EXAMPLE 25

### Construction of Vector for Coexpression of CRPV L1/L2 from a Single Plasmid

Plasmid pSP72-CRPV-L1 (p12930-314-4-1) was digested with BglII and the 1.5 kbp BglII fragment bearing the CRPV L1 ORF with a yeast 5'-nontranslated leader was gel-purified. Plasmid p12930-323-2-3 was digested with BamHI which cuts between the *GAL1* promoter and the *ADH1* transcriptional terminator. The linear vector fragment was gel-purified and then ligated with the aforementioned CRPV-L1 BglII fragment to yield the plasmid p12930-366-1-2. This resulting plasmid contains the CRPV-L1 ORF under control of the *GAL1* promoter and the CRPV-L2 ORF under control of the *GAL10* promoter.

### EXAMPLE 26

### Construction of Vector for Coexpression of CRPV L1/L2 from Two Plasmids

The pUC18-GAL1p-GAL10p vector containing the L2 gene was cut with SphI and the 2.9 kb fragment harboring the ADH1t-GAL1p-GAL10p-L2-ADH1t expression cassette was gel-purified and made blunt-ended by treatment with T4 DNA polymerase. The yeast shuttle vector YEp24 [Botstein et al., Gene 8:17 (1979)] was digested with BamHI, made blunt-ended by treatment with T4 DNA polymerase, dephosphory-lated with calf intestine alkaline phosphatase and then ligated with the above blunt-ended L2 expression cassette to yield the plasmid p1594.

### EXAMPLE 27

### Coexpression of CRPV L1 and L2 in Yeast

Plasmid p12930-366-1-2 (pC1/1-GAL1/10p-CRPV/L1+L2) was used to transform S. cerevisiae strains #1569 and BJ5462 (one plasmid system) and the resulting transformants were selected on leucine-minus synthetic agar medium. In a parallel experiment, strain #1592 was cotransformed with CRPV-L1 expression vector p12930-323-6-1 plus the YEp24-GAL10p-L2 expression vector p1594 (two plasmid system) and the resulting transformants containing both vectors were selected on synthetic agar medium lacking both leucine and uracil. Clonal isolates for both the one plasmid system and two plasmid system were grown at 30°C in YEHD complex medium containing 2% galactose for 48-72 hours. After harvesting the cells, the cell pellets were broken with glass beads. TritonX-100 was added to 0.5% final concentration and cell lysates were analyzed for the expression of CRPV L1 and L2 by immunoblot analysis. Samples containing 50 µg of total cellular protein were electrophoresed on 8-16% Tris-Glycine gradient gels (Novex) under reducing and denaturing conditions and electroblotted onto PVDF membranes (Novex). CRPV L1 and L2 proteins were detected using polyclonal rabbit anti-L1 or anti-L2 antisera (gift of Dr. John Kreider, Hershey Medical Center) as primary antibodies and protein A coupled to horseradish peroxidase (Amersham, Inc.) as the secondary antibody. The membranes were processed using the chemiluminescent ECL™ Detection Kit (Amersham, Inc.). A 55-61 kDa L1 protein band and a ∼90 kDa L2 protein band was detected in all samples from yeast clones harboring the L1 + L2 expression plasmid. No signal for L2 was detected in samples derived from yeast clones harboring the expression plasmid for L1 (Figure 6). No signal for L1 was detected in samples from cells containing only the L2 expression vector.

### EXAMPLE 28

### Purification of CRPV L1 and CRPV L1 + L2 VLPs for EM studies

The yeast expressed CRPV L1 and CRPV L1 and L2 proteins were partially purified and concentrated for electron microscopy (EM) studies. One to 1.5 liters of YEHD medium containing 2% galactose were inoculated with S. cerevisiae strain #1569 or BJ5462 transformed with the L1 + L2 coexpression vector p12930-366-1-2 and grown at 30°C for 48-72 hours. In a parallel experiment, cells of strain #1569 co-transformed with the CRPV-L1 expression vector p12930-323-6-1 plus the YEp24-GAL10p-L2 plasmid p1594 were grown in a similar fashion. The cells were harvested and cell pellets frozen at -70°C. All following steps were performed at 4°C. Cell pellets were thawed and suspended in an equal volume of "L1 buffer" (20 mM sodium phosphate, pH 7.2, 100 mM NaCl, 1.7 mM EDTA). Protein inhibitors PMSF and Pepstatin A were added to the slurry at a final concentration of 2 µM and 1.7 µM respectively. Cells were lysed by 3-5 passages in a microfluidizer. The lysates were clarified by centrifugation at 5000 x g for 10 min. The supernatant was layered on top of a 5 cm cushion of 45% (v/v) sucrose in L1 buffer and the L1, L2 or L1 and L2 proteins were pelleted by centrifugation at 100,000 x g for 4 hours. The pellet was resuspended in 1/10th the volume of L1 buffer. The resuspended pellet was clarified by centrifugation at 5000 x g for 10 min.

For EM analysis (Structure Probe, West Chester, PA), an aliquot of each sample was placed on 200 mesh carbon coated copper grids. A drop of 2% phosphotungstic acid (PTA), pH 7.0 was placed on the grid for 20 seconds. The grids were allowed to air dry prior to TEM examination. All microscopy was done using a JEOL 100CX transmission electron microscope (JEOL USA, Inc.) at an accelerating voltage of 100 KV. The micrographs generated have a final magnification of 100,000X.

In all yeast samples harboring either the CRPV L1 expression plasmid or plasmids for the co-expression of CRPV L1 and L2, VLPs were observed ≤25 nm diameter size range. No VLP were observed in yeast control samples or yeast samples harboring the L2 expression plasmid alone.

### EXAMPLE 29

### Expression of CRPV L1 (strain 1582) and HPV Type 6a L1 (strain 1644) in enriched complex and chemically-defined media.

Inocula for these strains were developed in leucine-free synthetic medium as described above for transfer to shake flask cultures. The shake flasks used were baffled for high aeration capacity (70 mL liquid per 300 mL flask, Tunair Labware) and media were supplemented with ca. 0.5 mL/L of an antifoam (UCON LB-625, Union Carbide). Enriched complex medium contained (per L): 40 g Difco yeast extract; 20 g Sheffield HySoy peptone; 30 g glucose; 50 g galactose; the medium was adjusted to pH 5.3 prior to sterilization. The chemically-defined medium used was similar to that described by Oura (Biotechnol. Bioengineer.16: 1197-1212. 1974) but supplemented with (per L): 0.1 g choline Cl; 0.4 g adenine; 30 g monosodium glutamate as nitrogen source; 0.2 g uracil; 20 g glucose; 40 g galactose. Flasks were inoculated with 3 mL inoculum and incubated for 66 hr at 28 C, 250 rpm on a rotary shaker. Flasks were sampled at intervals for verification of expression by immunoblot using anti-CRPV L1 and anti-HPV 6a L1 antisera.

### EXAMPLE 30

### Cloning of HPV16 L1 and L2 Genes

Total genomic DNA was extracted from Caski cells (ATCC #CRL 1550) by standard techniques (Sambrook et al., supra). The DNA was digested with Bstl 107I and SphI endonucleases and electrophoresed through a 0.8% low-melting-temperature agarose preparative gel. A region corresponding to DNA of ∼3.5-kbp was excised from the gel and the agarose was digested with Gelase™ enzyme (Epicentre Technologies, Inc.). The gel-purified DNA was made blunt-ended with T4 DNA polymerase and ligated with blunt-ended, phosphorylated oligodeoxynucleotide linkers that contain a buried HindIII site. The ligation mixture was digested to completion with HindIII and the ∼3.5-kbp DNA was size-fractionated through an agarose gel as described above. The gel-purified DNA was ligated with pUC18 plasmid DNA that had been digested with HindIII and dephosphorylated. Following transformation of competent *E.coli* DH5 cells (BRL), the plasmid library was screened for HPV16-positive clones by colony hybridization using an antisense ³²P-labeled oligodeoxynucleotide that is complementary to the 3'-end of the HPV-16 L1 gene (5'-GAG AGA TCT TAC AGC TTA CGT TTT TTG CGT TTA GC-3'). A pUC18 plasmid containing a 3.3-kbp HPV16 genomic fragment was isolated and characterized by restriction enzyme and Southern blot analyses. This plasmid was designated pUC18-HPV16 L1/L2 and contains all of the L1 and L2 coding DNA sequences. Plasmid DNA was prepared using the Qiagen™ Plasmid Maxi kit (Qiagen, Inc.).

### EXAMPLE 31

### Construction of HPV16 L1 Yeast Expression Vector

The clone, pUC18-HPV16 L1/L2 was used as a template for PCR. The HPV16 L1 gene was amplified by PCR using Vent polymerase (New England Biolabs, Inc.), 10 cycles of amplification (94°C, 1 min; 48°C, 1 min; 72°C, 1 min 45 sec), and the following oligodeoxynucleotide primers which contain flanking BglII sites (underlined):

The sense primer introduces a yeast non-translated leader sequence immediately upstream to the HPV16 L1 initiating methionine codon (highlighted in bold print). The 1.5-kbp L1 PCR product was digested with BglII, gel-purified, and ligated with the BamHI digested pGAL10 vector. A pGAL10 plasmid was isolated containing the HPVI6 L1 gene and designated, p14049-37-1. The L1 gene in p14049-37-1 was sequenced using the PRISM™ kit (ABI, Inc.) and an ABI Sequencer Model #373A according to the manufacturer's directions. The L1 gene in this isolate was shown to contain 3 nucleotide changes from the corrected, published prototype sequence (Kirnbauer, R. et al. (1993) J. Virol. 67: 6929-6936), resulting in two amino acid changes: His-202 to Asp; Thr-266 to Ala. Sequence analysis of the original template DNA confirmed that these changes were also present in the genomic clone pUC18-HPV16 L1/L2 and were not introduced by the PCR.

### EXAMPLE 32

### Construction of the HPV16 L1 and L2 Yeast Expression Vector

Plasmid p14049-37-1 was digested with Smal, which cuts between the *GAL10* promoter and the *ADH1* transcription terminator. The 1.4-kbp HPV16 L2 gene was amplified by PCR using the pUC18-HPV16 L1/L2 DNA as template, Vent polymerase (New England Biolabs, Inc.), 10 cycles of PCR amplification (94°C, 1 min; 48°C, 1 min; 72°C, 1 min 45 sec) and the following oligodeoxynucleotide primers which contain flanking SmaI sites (underlined):

The sense primer introduces a yeast non-translated leader sequence immediately upstream to the HPV16 L2 initiating methionine codon (highlighted in bold print). The 1.4-kbp L2 PCR product was digested with Smal, gel-purified, and ligated with the SmaI digested p14049-37-1 vector. A pLS110 plasmid containing both the HPV16 L1 and L2 genes was isolated and designated, p14049-42-2. The L2 gene in p14049-42-2 was sequenced using the PRISM™ kit (ABI, Inc.) and an ABI Sequencer Model #373A according to the manufacturer's directions. The L2 gene in this isolate was shown to contain 5 nucleotide changes from the corrected, published prototype sequence (Kimbauer, R. et al. (1993) *supra*), resulting in one amino acid change: Ser-269 to Pro. Sequence analysis of the genomic clone pUC18-HPV16 L1/L2 confirmed that this change was also present in the original template DNA and was not introduced by the PCR.

### EXAMPLE 33 (Reference)

### A. Expression of HPV16 L1 and Co-Expression of HPV16 L1 and L2 in Yeast

Plasmids p14049-37-1 and P14049-42-2 were used to transform *S. cerevisiae* strain #1558. The resulting recombinant strains were #1678 (HPV16-L1) and #1679 (HPV16 L1+L2) as shown in the table. Clonal isolates were grown at 30°C in YEHD medium containing 2% galactose for 68-78 hours. After harvesting the cells, the cell pellets were broken with glass beads and cell lysates analyzed for the expression of HPV16 L1 or HPV 16 L2 protein by immunoblot analysis. Samples containing 40 mcg of total cellular protein were electrophoresed on 10% Tris-Glycine gels under reducing and denaturing conditions and electroblotted onto nitrocellulose filters. The HPV16L1 protein was immunodetected using rabbit polyclonal antisera raised against a trpE-HPV11 L1 fusion protein (D. Brown et al., Virology 201:46-54) as primary antibody and HRP-linked, donkey anti-rabbit IgG antibody (Amersham, Inc.) as the secondary antibody. The filters were processed using the chemiluminescent ECL™ Detection kit (Amersham, Inc.). A 50-55 kDa L1 protein band was detected in all samples except the negative control (vector control without L1 or L2 gene). The L2 protein was detected as a 70 kDa protein band by immunoblot using mouse anti-HPV16 L2 sera raised against trpE-L2 fusion proteins expressed in E. *coli* as primary antibody. Goat anti-mouse IgG HRP-linked (Amersham, Inc.) was used as secondary antibody and the filters were processed as described above.

### B. Purification of Recombinant HPV Type 16 L1 + L2 Capsid Proteins

All steps performed at 4 ° C unless noted.

Cells were stored frozen at -70 ° C. Frozen cells (wet weight = 27.6 g) were thawed at 20-23 ° C and resuspended in 40 mL "L1 Buffer" (20 mM sodium phosphate, pH 7.2, 100 mM NaCl, 1.7 mM EDTA). The protease inhibitors PMSF and pepstatin A were added to final concentrations of 2 mM and 1.7 mcM, respectively. The cell slurry was broken at a pressure of approximately 8,000 psi by 3 passes in a M110 Microfluidizer (Microfluidics Corp., Newton, MA). The broken cell slurry was centrifuged at 5,000 x g for 10 min to remove cellular debris. The supernatant liquid containing L1 + L2 antigen was recovered.

The supernatant liquid was diluted 1:5 by addition of Buffer A (20 mM MOPS, pH 7.0) and applied to an anion exchange capture column (5.0 cm ID x 4.8 cm) of Fractogel® EMD TMAE-650 (S) resin (EM Separations, Gibbstown, NJ) equilibrated in Buffer A. Following a wash with Buffer A, the antigen was eluted with a gradient of 0 - 1.0 M NaCl in Buffer A. Immuno-dot blotting was performed to determine which fractions from the column contained L1 protein.

Fractions containing L1 protein as determined by immuno-dot blot were assayed for total protein by the Bradford method followed by SDS-PAGE using silver staining and Western blotting.

TMAE fractions showing comparable purity and enrichment of L1 protein were pooled. The antigen was concentrated by ammonium sulfate fractionation. Samples were adjusted to 48% saturated ammonium sulfate by addition of solid reagent while gently stirring over 30 min. The samples were placed on ice and precipitation allowed to proceed overnight. The samples were centrifuged at 12,000 x g. The pellet was resuspended in 20.0 mL PBS (6.25 mM Na phosphate, pH 7.2, 150 mM NaCl).

Resuspended pellets were chromatographed separately on a size-exclusion column (2.6 cm ID x 89 cm) of Sephacryl 500 HR resin (Pharmacia, Piscataway, NJ) at 20-23 ° C. Running buffer was PBS. Fractions were analyzed by SDS-PAGE using silver staining and western blot detection. The purest fractions were pooled. Resulting pools were concentrated in a 50 mL stirred cell using 43 mm YM-100 flat-sheet membranes (Amicon, Beverly, MA) at a N2 pressure of 4-6 psi.

Final product was analyzed by SDS-PAGE using colloidal Coomassie staining. The L1 and L2 proteins were estimated to be 70% homogeneous. The identity of L1 and L2 proteins was confirmed by western blotting using the appropriate antisera. The final product was aliquoted and stored at -70 ° C. This process resulted in a total of 0.53 mg protein.

Electron microscopy analysis was performed by Structure Probe (West Chester, PA). An aliquot of sample was placed on a 200 mesh carbon-coated copper grid. A drop of 2% phosphotungstic acid, pH 7.0 was placed on the grid for 20 seconds. The grid was allowed to air dry prior to TEM examination. All microscopy was performed using a JEOL 100 CX transmission electron microscope (JEOL USA, Inc.) at an accelerating voltage of 100 kV. The micrographs generated have a final magnification of 100,000 x. The presence of virus-like particles in the ≤25 nm size range was confirmed.

### C. Purification of Recombinant HPV Type 16 L1 + L2 Capsid Proteins

All steps performed at 4 °C unless noted.

Cells were stored frozen at -70 ° C. Frozen cells (wet weight = 92.8 g) were thawed at 20-23 ° C and resuspended in 105 mL "L1 Buffer" (20 mM sodium phosphate, pH 7.2, 100 mM NaCl, 1.7 mM EDTA). The protease inhibitors PMSF and pepstatin A were added to final concentrations of 2 mM and 1.7 mcM, respectively. The cell slurry was broken at a pressure of approximately 16,000 psi by 3 passes in a M110-Y Microfluidizer (Microfluidics Corp., Newton, MA). The broken cell slurry was centrifuged at 6,100 x g for 15 min to remove cellular debris. The supernatant liquid containing L1 + L2 antigen was recovered.

The supernatant liquid was diluted 1:5 by addition of Buffer A (20 mM MOPS, pH 7.0) and applied to an anion exchange capture column (5.0 cm ID x 4.2 cm) of Fractogel ® EMD TMAE-650 (S) resin (EM Separations, Gibbstown, NJ) equilibrated in Buffer A. Following a wash with Buffer A, the antigen was eluted with a gradient of 0 - 1.0 M NaCl in Buffer A. Immuno-dot blotting was performed to determine which fractions from the column contained L1 protein.

Fractions containing L1 protein as determined by immuno-dot blot were assayed for total protein by the Bradford method followed by SDS-PAGE using silver staining and Western blotting.

TMAE fractions showing comparable purity and enrichment of L protein were pooled. The antigen was concentrated by ammonium sulfate fractionation. Samples was adjusted to 35% saturated ammonium sulfate by addition of solid reagent while gently stirring over 10 min. The samples were placed on ice and precipitation allowed to proceed for 4 hours. The samples were centrifuged at 12,000 x g. The pellet was resuspended in 20.0 mL PBS (6.25 mM Na phosphate, pH 7.2, 150 mM NaCI) which contained 1 mM EDTA.

Resuspended pellets were chromatographed separately on a size-exclusion column (2.6 cm ID x 89 cm) of Sephacryl 500 HR resin (Pharmacia, Piscataway, NJ). Running buffer was PBS + 1mM EDTA. Fractions were analyzed by SDS-PAGE using silver staining and western blot detection. The purest fractions were pooled. Resulting pools were concentrated in a 50 mL stirred cell using 43 mm YM-100 flat-sheet membranes (Amicon, Beverly, MA) at a N₂ pressure of 4-6 psi.

Final product was analyzed by SDS-PAGE using colloidal Coomassie staining. The L1 and L2 proteins were estimated to be 70% homogeneous. The identity of L1 and L2 proteins was confirmed by western blotting using the appropriate antisera. The final product was aliquoted and stored at -70 ° C. This process resulted in a total of 3.8 mg protein.

### EXAMPLE 34 (Reference)

### A. Fermentation of Strain 1679 (HPV type 16 L1+L2)

Procedures used for preparation frozen stock cultures, inoculum development, fermentation, and cell recovery of strain 1679 were essentially as described above. After 67 hr incubation, a cell density of 4.2 g dry cell weight per L was reached and yielded a total of 93 g wet cell pellet after recovery.

### B. Fermentation of Strain 1678 (HPV type 16 L1).

Procedures used for preparation frozen stock cultures, inoculum development, fermentation, and cell recovery of strain 1678 were essentially as described above. After 70.5 hr incubation, the contents of two 10 L fermentations were pooled (a cell density of 8.7 g dry cell weight per L) which yielded a total of 258 g wet cell pellet after recovery.

### C. Purification of Recombinant HPV Type 16 L 1 Capsid Proteins

All steps performed at 4 ° C unless noted.

Cells of strain #1678 were stored frozen at -70 ° C. Frozen cells (wet weight = 128 g) were thawed at 20-23 ° C and resuspended in 140 mL "Modified L1 Buffer" (20 mM sodium phosphate, pH 7.2, 100 mM NaCl). The protease inhibitors PMSF and pepstatin A were added to final concentrations of 2 mM and 1.7 mcM, respectively. The cell slurry was broken at a pressure of approximately 16,000 psi by 3 passes in a M110-Y Microfluidizer (Microfluidics Corp., Newton, MA). The broken cell slurry was centrifuged at 11,000 x g for 40 min to remove cellular debris. The supernatant liquid containing L1 antigen was recovered.

The supernatant liquid was diluted 1:5 by addition of Buffer A (20 mM MOPS, pH 7.0) and applied to an anion exchange capture column (5.0 cm ID x 6.4 cm) of Fractogel ® EMD TMAE-650 (S) resin (EM Separations, Gibbstown, NJ) equilibrated in Buffer A. Following a wash with Buffer A, the antigen was eluted with a gradient of 0 - 1.0 M NaCl in Buffer A. Immuno-dot blotting was performed to determine which fractions from the column contained L1 protein.

Fractions containing L1 protein as determined by immuno-dot blot were assayed for total protein by the Bradford method followed by SDS-PAGE using silver staining and Western blotting.

TMAE fractions showing comparable purity and enrichment of L1 protein were pooled. The antigen was concentrated by ammonium sulfate fractionation. Samples were adjusted to 35% saturated ammonium sulfate by addition of solid reagent while gently stirring over 10 min. The samples were placed on ice and precipitation allowed to proceed for 5 hours. The samples were centrifuged at 12,000 x g. The pellet was resuspended in 20.0 mL PBS (6.25 mM Na phosphate, pH 7.2, 150 mM NaCl).

Resuspended pellets were chromatographed separately on a size-exclusion column (2.6 cm ID x 89 cm) of Sephacryl 500 HR resin (Pharmacia, Piscataway, NJ). Running buffer was PBS. Fractions were analyzed by SDS-PAGE using silver staining and western blot detection. The purest fractions were pooled. Resulting pools were concentrated in a 50 mL stirred cell using 43 mm YM-100 flat-sheet membranes (Amicon, Beverly, MA) at a N2 pressure of 4-6 psi.

Final product was analyzed by SDS-PAGE using colloidal Coomassie staining. The L1 protein was estimated to be 70% homogeneous. The identity of L1 was confirmed by western blotting using the appropriate antisera. The final product was aliquoted and stored at -70 ° C. This process resulted in a total of 7.4 mg protein.

### D. Analytical Procedures

### Immuno-Dot Blot Procedure

Samples were diluted (when necessary) 1:10 in Milli-Q-H₂O and 10 mcL of sample was spotted onto PolyScreen™ PVDF membranes (NEN Research Products, Boston, MA). After spots had dried, the membranes were washed in water and allowed to dry. Primary antibody solution was prepared by dilution of the appropriate antiserum in blotting buffer (5% non-fat milk in 6.25 mM Na phosphate, pH 7.2, 150 mM NaCI, 0.02% NaN₃). Incubation was for at least 1 hour at 20-23 ° C. The blot was washed for 1 min each in three changes of PBS (6.25 mM Na phosphate, pH 7.2, 150 mM NaCl). Secondary antibody solution was prepared by diluting the appropriate alkaline phosphatase-linked conjugate antiserum in blotting buffer. Incubation proceeded under the same conditions for at least 1 hour. Blots were washed as before and detected using a 1 step NBT/BCIP substrate (Pierce, Rockford, IL).

Antibodies used for detection were as follows:
HPV 6aLl was detected by MAB 837 (Chemicon International, Inc., Temecula, CA). HPV 6a L2 was detected by mouse anti-HPV6a L2-trpE fusion serum pool # 641 and 647 (produced in-house by M. Rosolowski). HPV 16 L1 was detected by MAB 885 (Chemicon International, Inc., Temecula, CA). HPV 16 L2 was detected by mouse anti-HPV16 L2-trpE fusion serum pool # 611 (produced in-house by K. Jansen).

### Bradford Assay for Total Protein

Total protein was assayed using a commercially available Coomassie Plus ® kit (Pierce, Rockford, IL). Samples were diluted to appropriate levels in Milli-Q-H₂O. Volumes required were 0.1 mL and 1.0 mL for the standard and microassay protocols, respectively. For both protocols, BSA (Pierce, Rockford, IL) was used to generate the standard curve. The assay was performed according to manufacturer's recommendations. Standard curves were plotted using CricketGraph ® software on a Macintosh IIci computer.

### SDS-PAGE and Western Blot Assays

All gels, buffers, and electrophoretic apparatus were obtained from Novex (San Diego, CA) and were run according to manufacturer's recommendations. Briefly, samples were diluted to equal protein concentrations in Milli-Q-H₂O and mixed 1:1 with sample incubation buffer containing 200 mM DTT. Samples were incubated 15 min at 100 ° C and loaded onto pre-cast 12% Tris-glycine gels. The samples were electrophoresed at 125V for 1 hr 45 min. Gels were developed using either silver staining by a variation of the method of Heukeshoven and Dernick [*Electrophoresis, 6 (1985) 103-112*] or colloidal Coomassie staining using a commercially obtained kit (Integrated Separation Systems, Natick, MA). For western blots, proteins were transferred to PVDF membranes at 25V for 40 min. Membranes were dried and incubated with antibody solutions as for immuno-dot blots.

### EXAMPLE 35

### Preparation of Immunogenic Compositions

Purified VLP's are formulated according to known methods, such as by the admixture of pharmaceutically acceptable carriers, stabilizers, or a vaccine adjuvant. The immunogenic VLP's of the present invention may be prepared for vaccine use by combining with a physiologically acceptable composition such as, e.g. PBS, saline or distilled water. The immunogenic VLP's are administered in a dosage range of about 0.1 to 100 mcg, preferably about 1 to about 20 mcg, in order to obtain the desired immunogenic effect. The amount of VLP per formulation may vary according to a variety of factors, including but not limited to the individual's condition, weight, age and sex. Administration of the VLP formulation may be by a variety of routes, including but not limited to oral, subcutaneous, topical, mucosal and intramuscular. Such VLP formulations may be comprised of a single type of VLP (i.e., VLP from HPV6a) or a mixture of VLP's (i.e, VLP's from HPV6a, HPV11, HPV16 and HPV18).

An antimicrobial preservative, e.g. thimerosal, optionally may be present. The immunogenic antigens of the present invention may be employed, if desired, in combination with vaccine stabilizers and vaccine adjuvants. Typical stabilizers are specific compounds, e.g. polyanions such as heparin, inositol hexasulfate, sulfated betacyclodextrin, less specific excipients, e.g. amino acids, sorbitol, mannitol, xylitol, glycerol, sucrose, dextrose, trehalose, and variations in solution conditions, e.g. neutral pH, high ionic strength (ca. 0.5-2.0 M salts), divalent cations (Ca²⁺, Mg²⁺). Examples of adjuvants are Al(OH)₃ and Al(PO₄). The vaccine of the present invention may be stored under refrigeration or in lyophilized form.

### EXAMPLE 36

### Preparation of Antibodies to VLP

Purified VLP are used to generate antibodies. The term "antibody" as used herein includes both polyclonal and monoclonal antibodies as well as fragments thereof, such as Fv, Fab and F(ab)2 fragments that are capable of binding antigen or hapten. The antibodies are used in a variety of ways, including but not limited to the purification of recombinant VLP, the purification of native L1 or L2 proteins, and kits. Kits would comprise a compartmentalized carrier suitable to hold in close confinement at least one container. The carrier would further comprise reagents such as the anti-VLP antibody or the VLP suitable for detecting HPV or fragments of HPV or antibodies to HPV. The carrier may also contain means for detection such as labeled antigen or enzyme substrates or the like. The antibodies or VLP or kits are useful for a variety of purposes, including but not limited to forensic analyses and epidemiological studies.

### EXAMPLE 37

### Purification of Recombinant HPV Type 11 L1 Capsid Proteins

All steps were performed at 4°C unless noted.

Cells were stored frozen at -70°C. Frozen cells (wet weight = 180 g) were thawed at 20-23°C and resuspended in 900 mL "Breaking Buffer" (50 mM MOPS, pH 7.2, 500 mM NaCl, 1 mM CaCl₂). The protease inhibitors AEBSF and pepstatin A were added to final concentrations of 1 mM and 1.7 µM, respectively. The cell slurry was broken at a pressure of approximately 16,000 psi by 4 passes in a M110-Y Microfluidizer (Microfluidics Corp., Newton, MA). A sufficient volume of 10% Triton X100® detergent (Pierce, Rockford, IL) was added to the broken cell slurry to bring the concentration of TX100 to 0.5%. The slurry was stirred for 20 hours. The Triton X100-treated lysate was centrifuged at 12,000 x g for 40 min to remove cellular debris. The supernatant liquid containing L1 protein was recovered.

The supernatant liquid was diafiltered against five volumes of 20 mM sodium phosphate, pH 7.2, 0.5 M NaCI using a 300K tangential flow membrane cassette (Filtron, Northborough, MA). The material retained by the membrane was shown by radioimmunoassay and western blotting to contain the L1 protein.

The retentate was applied to a high resolution affinity column (11.0 cm ID x 5.3 cm) of SP Spherodex (M)® resin (IBF, Villeneuve-la-Garenne, France) equilibrated in 20 mM sodium phosphate, pH 7.2, 0.5 M NaCl. Following a wash with equilibration buffer and a step wash with 20 mM sodium phosphate, pH 7.2, 1.0 M NaCl, the L1 protein was eluted with a step wash of 20 mM sodium phosphate, pH 7.2, 2.5 M NaCl. Fractions were collected during the washes and elution. Column fractions were assayed for total protein by the Bradford method. Fractions were then analyzed by western blotting and SDS-PAGE with colloidal Coomassie detection. Fractions were also analyzed by radioimmunoassay.

SP Spherodex fractions showing comparable purity and enrichment of L1 protein were pooled.

Final product was analyzed by western blotting and SDS-PAGE with colloidal Coomassie detection. The L1 protein was estimated to be ≥ 90% homogeneous. The identity of L1 protein was confirmed by western blotting. The final product was filtered aseptically through a 0.22 µm membrane and stored at 4°C. This process resulted in a total of 100 mg protein.

Electron microscopy analysis is performed by Structure Probe (West Chester, PA). An aliquot of sample is placed on a 200 mesh carbon-coated copper grid. A drop of 2% phosphotungstic acid, pH 7.0 is placed on the grid for 20 seconds. The grid is allowed to air dry prior to TEM examination. All microscopy is performed using a JEOL 100 CX transmission electron microscope (JEOL USA, Inc.) at an accelerating voltage of 100 kV. The micrographs generated have a final magnification of 100,000 x.

### Bradford Assay for Total Protein

Total protein was assayed using a commercially available Coomassie Plus® kit (Pierce, Rockford, IL). Samples were diluted to appropriate levels in Milli-Q-H₂O. Volumes required were 0.1 mL and 1.0 mL for the standard and microassay protocols, respectively. For both protocols, BSA (Pierce, Rockford, IL) was used to generate the standard curve. Assay was performed according to manufacturer's recommendations. Standard curves were plotted using CricketGraph® software on a Macintosh IIci computer.

### SDS-PAGE and Western Blot Assays

All gels, buffers, and electrophoretic apparatus were obtained from Novex (San Diego, CA) and were run according to manufacturer's recommendations. Briefly, samples were diluted to equal protein concentrations in Milli-Q-H₂O and mixed 1:1 with sample incubation buffer containing 200 mM DTT. Samples were incubated 15 min at 100°C and loaded onto pre-cast 12% Tris-glycine gels. The samples were electrophoresed at 125V for 1 hr 45 min. Gels were developed by colloidal Coomassie staining using a commercially obtained kit (Integrated Separation Systems, Natick, MA).

For western blots, proteins were transferred to PVDF membranes at 25V for 40 min. Membranes were washed with Milli-Q-H2O and air-dried. Primary antibody was polyclonal rabbit antiserum raised against a TrpE-HPV11L1 fusion protein (gift of Dr. D. Brown). The antibody solution was prepared by dilution of antiserum in blotting buffer (5% non-fat milk in 6.25 mM Na phosphate, pH 7.2, 150 mM NaCl, 0.02% NaN₃). Incubation was for at least 1 hour at 20-23°C. The blot was washed for 1 min each in three changes of PBS (6.25 mM Na phosphate, pH 7.2, 150 mM NaCI). Secondary antibody solution was prepared by diluting goat anti-rabbit IgG alkaline phosphatase-linked conjugate antiserum (Pierce, Rockford, IL) in blotting buffer. Incubation proceeded under the same conditions for at least 1 hour. Blots were washed as before and detected using a 1 step NBT/BCIP substrate (Pierce, Rockford, IL).

### EXAMPLE 38

### Purification of Recombinant HPV Type 6a L1 Capsid Proteins

All steps performed at 4°C unless noted.

Cells were stored frozen at -70°C. Frozen cells (wet weight = 60 g) were thawed in a 45°C water bath and resuspended in 300 mL "Breaking Buffer" (50 mM MOPS, pH 7.2, 500 mM NaCl, 1 mM CaCl₂). The protease inhibitors AEBSF and pepstatin A were added to final concentrations of 1 mM and 1.7 µM, respectively. The cell slurry was broken at a pressure of approximately 16,000 psi by 5 passes in a M110-Y Microfluidizer (Microfluidics Corp., Newton, MA). A sufficient volume of 10% Triton X100® detergent (Pierce, Rockford, IL) was added to the broken cell slurry to bring the concentration of TX100 to 0.5%. The slurry was stirred for 18 hours. The Triton X100-treated lysate was centrifuged at 12,000 x g for 40 min to remove cellular debris. The supernatant liquid containing L1 protein was recovered.

The supernatant liquid was diafiltered against five volumes of 20 mM sodium phosphate, pH 7.2, 0.5 M NaCl using a 300K tangential flow membrane cassette (Filtron, Northborough, MA). The material retained by the membrane was shown by radioimmunoassay and western blotting to contain ≥ 90% of the original amount of L1 protein.

The retentate was applied to a high resolution column (5.0 cm ID x 10.0 cm) of POROS ® 50HS resin (Perseptive Biosystems, Cambridge, MA) equilibrated in 20 mM sodium phosphate, pH 7.2, 0.5 M NaCl. Following a wash with equilibration buffer, the L1 protein was eluted with a linear gradient of 0.5-1.5 M NaCl in 20 mM sodium phosphate, pH 7.2. Fractions were collected during the washes and elution. Column fractions were assayed for total protein by the Bradford method. Fractions were then analyzed by western blotting and SDS-PAGE with colloidal Coomassie detection. Fractions were also analyzed by radioimmunoassay.

Column fractions showing comparable purity and enrichment of L1 protein were pooled. The pooled fractions were filtered aseptically through a 0.22 µm membrane. Product was concentrated in a 50 mL stirred cell using 43 mm YM-100 flat-sheet membranes (Amicon, Beverly, MA) at a N2 pressure of 4-6 psi. Product was stored at 4 ° C. This process resulted in a total of 2.7 mg protein.

A sample of final product was further concentrated by TCA precipitation and analyzed by western blotting and SDS-PAGE with colloidal Coomassie detection. The L1 protein was shown by densitometry of colloidal Coomassie stained gels to be ≥ 90% homogeneous. The identity of L1 protein was confirmed by western blotting.

Electron microscopy analysis is performed by Structure Probe (West Chester, PA). An aliquot of sample is placed on a 200 mesh carbon-coated copper grid. A drop of 2% phosphotungstic acid, pH 7.0 is placed on the grid for 20 seconds. The grid is allowed to air dry prior to TEM examination. All microscopy is performed using a JEOL 100 CX transmission electron microscope (JEOL USA, Inc.) at an accelerating voltage of 100 kV. The micrographs generated have a final magnification of 100,000 x.

### Bradford Assa for Total Protein

Total protein was assayed using a commercially available Coomassie Plus® kit (Pierce, Rockford, IL). Samples were diluted to appropriate levels in Milli-Q-H₂O. Volumes required were 0.1 mL and 1.0 mL for the standard and microassay protocols, respectively. For both protocols, BSA (Pierce, Rockford, IL) was used to generate the standard curve. Assay was performed according to manufacturer's recommendations. Standard curves were plotted using CricketGraph® software on a Macintosh IIci computer.

### SDS-PAGE and Western Blot Assays

All gels, buffers, and electrophoretic apparatus were obtained from Novex (San Diego, CA) and were run according to manufacturer's recommendations. Briefly, samples were diluted to equal protein concentrations in Milli-Q-H20 and mixed 1:1 with sample incubation buffer containing 200 mM DTT. Samples were incubated 15 min at 100°C and loaded onto pre-cast 12% Tris-glycine gels. The samples were electrophoresed at 125V for 1 hr 45 min. Gels were developed by colloidal Coomassie staining using a commercially obtained kit (Integrated Separation Systems, Natick, MA).

For western blots, proteins were transferred to PVDF membranes at 25V for 40 min. Membranes were washed with Milli-Q-H2O and air-dried. Primary antibody was MAB 837 (Chemicon International, Inc., Temecula, CA) which had been covalently linked to alkaline phosphatase enzyme (J. Cook, MRL). The antibody solution was prepared by dilution of antiserum in blotting buffer (5% non-fat milk in 6.25 mM Na phosphate, pH 7.2, 150 mM NaCI, 0.02% NaN₃). Incubation was for at least 1 hour at 20-23°C. The blot was washed for 1 min each in three changes of PBS (6.25 mM Na phosphate, pH 7.2, 150 mM NaCI). Blots were detected using a 1 step NBT/BCIP substrate (Pierce, Rockford, IL).

## Claims

1. A method of producing a purified papillomavirus capsid protein comprising:
(a) transforming a yeast host with a recombinant DNA molecule, the recombinant DNA molecule encoding a protein selected from papillomavirus L1 protein and papillomavirus L1 + L2 proteins;
(b) cultivating the transformed host under conditions that permit expression of the recombinant DNA molecule to produce a crude recombinant papillomavirus protein; and
(c) purifying the crude recombinant papillomavirus protein with a single chromatography step using ion exchange chromatography to produce the purified protein, the protein being at least 90% pure.

2. A method according to claim 1 wherein the yeast is Saccharomyces cerevisiae.

3. A method according to claim 1 or 2 wherein the ion exchange chromatography is over POROS® resin.

4. A method according to claim 1, 2 or 3 wherein the papillomavirus is selected from human papillomavirus and cottontail rabbit papillomavirus.

5. A method according to claim 4 wherein the human papillomavirus is selected from HPV6a, HPV6b, HPV11, HPV16, HPV18, HPV31, HPV33, HPV35, HPV41, HPV45 and HPV58.

## Patentansprüche

1. Verfahren zur Herstellung eines gereinigten Papillomavirus-Kapsidproteins, umfassend:
(a) Transformation eines Hefewirts mit einem rekombinanten DNA-Molekül, wobei das rekombinante DNA-Molekül für ein Protein kodiert, das aus Papillomavirus-L1-Protein und Papillomavirus-L1+L2-Proteinen ausgewählt ist;
(b) Kultivierung des transformierten Wirts unter Bedingungen, welche die Expression des rekombinanten DNA-Moleküls erlauben, um ein rohes rekombinantes Papillomavirus-Protein herzustellen; und
(c) Reinigung des rohen rekombinanten Papillomavirus-Proteins mit einem einzigen Chromatographieschritt unter Anwendung von Ionenaustauschchromatographie, um das gereinigte Protein herzustellen, wobei das Protein zu mindestens 90 % rein ist.

2. Verfahren nach Anspruch 1, wobei die Hefe Saccharomyces cerevisae ist.

3. Verfahren nach Anspruch 1 oder 2, wobei die lonenaustauschchromatographie über POROS®-Harz erfolgt.

4. Verfahren nach Anspruch 1, 2 oder 3, wobei das Papillomavirus aus Human-Papillomavirus und Baumwollschwanzkaninchen-Papillomavirus ausgewählt ist.

5. Verfahren nach Anspruch 4, wobei das Human-Papillomavirus aus HPV6a, HPV6b, HPV11, HPV16, HPV18, HPV31, HPV33, HPV35, HPV41, HPV45 und HPV58 ausgewählt ist.

## Revendications

1. Procédé de production d'une protéine capsidique de papillomavirus purifiée comprenant :
(a) la transformation d'un hôte de type levure avec une molécule d'ADN recombinant, la molécule d'ADN recombinant codant pour une protéine choisie parmi la protéine L1 du papillomavirus et les protéines L1+L2 du papillomavirus
(b) la mise en culture de l'hôte transformé dans des conditions permettant l'expression de la molécule d'ADN recombinant en vue de produire une protéine de papillomavirus recombinante brute ; et
(c) la purification de la protéine de papillomavirus recombinante brute par une seule étape de chromatographie en utilisant une chromatographie échangeuse d'ions pour produire la protéine purifiée, la protéine ayant une pureté d'au moins 90%.

2. Procédé selon la revendication 1, dans lequel la levure est Saccharomyces cerevisiae.

3. Procédé selon la revendication 1, dans lequel la chromatographie échangeuse d'ions est conduite sur une résine POROS®.

4. Procédé selon la revendication 1, 2 ou 3, dans lequel le papillomavirus est choisi parmi le papillomavirus humain et le papillomavirus de lapin de garenne.

5. Procédé selon la revendication 4, dans lequel le papillomavirus est choisi parmi HPV6a, HPV6b, HPV11, HPV16, HPV18, HPV31, HPV33, HPV35, HPV41, HPV45 et HPV58.


### SEQUENCE LISTING

(1) GENERAL INFORMATION:
   (i) APPLICANT:
      Lehman, Ernest D.
      Cook, III James C.
      George, Hugh A.
      Hofmann, Kathryn J.
      Jansen, Kathrin U.
      Joyce, Joseph G.
      Markus, Henry Z.
      Schultz, Loren D.
   (ii) TITLE OF INVENTION: PURIFIED PAPILLOMAVIRUS PROTEINS
   (iii) NUMBER OF SEQUENCES: 20
   (iv) CORRESPONDENCE ADDRESS:
      (A) ADDRESSEE: Christine E. Carty
      (B) STREET: 126 E. Lincoln Avenue, P.O. Box 2000
      (C) CITY: Rahway
      (D) STATE: New Jersey
      (E) COUNTRY: USA
      (F) ZIP: 07065-0907
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US 08/339,368
      (B) FILING DATE: 14-NOV-1994
      (C) CLASSIFICATION:
   (viii) ATTORNEY/AGENT INFORMATION:
      (A) NAME: Carty, Christine E.
      (B) REGISTRATION NUMBER: 36,099
      (C) REFERENCE/DOCKET NUMBER: 19341Y
   (ix) TELECOMMUNICATION INFORMATION:
      (A) TELEPHONE: (908)594-6734
      (B) TELEFAX: (908)594-4720
(2) INFORMATION FOR SEQ ID NO:1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   CTTAAAGCTT ATGTCACTTT CTCTTGTATC                    30
(2) INFORMATION FOR SEQ ID NO:2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
   TGATAAGCTT GCTCAATGGT TCTCTTCCTC                    30
(2) INFORMATION FOR SEQ ID NO:3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
   TGGTCATCCC AAATCTTGAA A                             21
(2) INFORMATION FOR SEQ ID NO:4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   CACCGTAGTG TTTGGAAGCG A                                                21
(2) INFORMATION FOR SEQ ID NO:5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   GAGGCTACCA GCGAGCCGGG C                                                21
(2) INFORMATION FOR SEQ ID NO:6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
   GGCCAGTGGG CCAACAGGTT C                                                21
(2) INFORMATION FOR SEQ ID NO:7:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
   GAGAGATCTT ACCTTTTAGT TTTGGCGCGC TTAC 34
(2) INFORMATION FOR SEQ ID NO:8:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
   CTCAGATCTC ACAAAACAAA ATGTGGCGGC CTAGCGACAG CACAG 45
(2) INFORMATION FOR SEQ ID NO:9:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   GAGAGATCTT ACCTTTTAGT TTTGGCGCGC TTAC 34
(2) INFORMATION FOR SEQ ID NO:10:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
   TCCCCCGGGC ACAAAACAAA ATGGCACATA GTAGGGCCCG ACGAC 45
(2) INFORMATION FOR SEQ ID NO:11:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   TCCCCCGGGC TAGGCCGCCA CATCTGAAAA AAATAAGG 38
(2) INFORMATION FOR SEQ ID NO:12:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   GAAGATCTTC AAAACAAAAT GGCAGTGTGG CTGTCTAC 38
(2) INFORMATION FOR SEQ ID NO:13:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   GAAGATCTTT ATTAAGTACG TCTCTTGCGT TTAG 34
(2) INFORMATION FOR SEQ ID NO:14:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   GGAATTCACA AAACAAAATG GTTGCACGGT CACGAAAAC 39
(2) INFORMATION FOR SEQ ID NO:15:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   GGAATTCTTA TTCTGCGTAG ACAGCCACAC TG 32
(2) INFORMATION FOR SEQ ID NO:16:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   GAGAGATCTT ACAGCTTACG TTTTTTGCGT TTAGC 35
(2) INFORMATION FOR SEQ ID NO:17:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   CTCAGATCTC ACAAAACAAA ATGTCTCTTT GGCTGCCTAT GTAGGCC 47
(2) INFORMATION FOR SEQ ID NO:18:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   GAGAGATCTT ACAGCTTACG TTTTTTGCGT TTAGC                    35
(2) INFORMATION FOR SEQ ID NO:19:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
   TCCCCCGGGC ACAAAACAAA ATGCGACACA AACGTTCTGC AAAAC         45
(2) INFORMATION FOR SEQ ID NO:20:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   TCCCCCGGGC TAGGCAGCCA AAGAGACATC TGA                      33